(19) Europäisches Patentamt
European Patent Office
Office européen des brevets

(11) EP 0 621 282 B1

(12) **FASCICULE DE BREVET EUROPEEN**

(45) Date de publication et mention de la délivrance du brevet: **09.07.1997 Bulletin 1997/28**

(51) Int. Cl.$^6$: **C07H 11/00**, C07H 15/04, C07H 13/08, C07H 3/10, A61K 31/70

(21) Numéro de dépôt: **94400879.6**

(22) Date de dépôt: **22.04.1994**

(54) **3-Désoxy oligosaccharides, leurs procédés de préparation et les compositions pharmaceutiques qui les contiennent**

3-Deoxyoligosacchariden, Verfahren zur ihrer Herstellung und diese enthaltende pharmazeutische Zusammensetzungen

3-Deoxyoligosaccharides, process for their preparation and pharmaceutical compositions containing them

(84) Etats contractants désignés:
**AT BE CH DE DK ES FR GB GR IE IT LI LU MC NL PT SE**

(30) Priorité: **22.04.1993 FR 9304769**

(43) Date de publication de la demande:
**26.10.1994 Bulletin 1994/43**

(73) Titulaires:
- **SANOFI**
  **75008 Paris (FR)**
- **Akzo Nobel N.V.**
  **6824 BM Arnhem (NL)**

(72) Inventeurs:
- **Petitou, Maurice**
  **F-75013 Paris (FR)**
- **Jaurand, Guy François Bernard**
  **F-78117 Chateaufort (FR)**
- **Van Boeckel, Constant Adriaan Anton**
  **NL-5345 LX Oss (NL)**

(74) Mandataire: **Le Guen, Gérard et al**
**CABINET LAVOIX**
**2, place d'Estienne d'Orves**
**75441 Paris Cédex 09 (FR)**

(56) Documents cités:
**EP-A- 0 454 220** **EP-A- 0 529 715**

- **ANGEWANDTE CHEMIE INTERNATIONAL EDITION IN ENGLISH, vol.32, no.3, Mars 1993, WEINHEIM, GERMANY pages 434 - 436 H. LUCAS ET AL 'A short synthetic route towards a biologically active heparin-like pentasaccharide with a pseudo-alternating sequence'**
- **ANGEWANDTE CHEMIE, Int. Ed. in Engl., vol. 32, no. 3, Mars 1993, pages 434-436, H. LUCAS et al.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.10/3.4

EP 0 621 282 B1

## Description

La présente invention concerne des 3-désoxy oligosaccharides, leurs procédés de préparation et les compositions pharmaceutiques qui les contiennent.

L'héparine est un polysaccharide de la famille des glycosaminoglycanes, connu pour ses propriétés anticoagulantes. Il est connu (I. Björk et U. Lindahl, "Molecular and Cellular Biochemistry", (1982), Dr. W. Junk Publishers - Pays-Bas) que la coagulation sanguine est un phénomène physiologique complexe. Certains stimuli, tels que l'activation de contact et les facteurs tissulaires, déclenchent l'activation successive d'une série de facteurs de coagulation présents dans le plasma sanguin. Quelle que soit la nature du stimulus, les étapes finales sont identiques le facteur X activé (Xa) active le facteur II (également appelé prothrombine), lequel sous sa forme activée (facteur IIa, également appelé thrombine) provoque la protéolyse partielle du fibrinogène soluble avec libération de la fibrine insoluble, constituant principal du caillot sanguin.

Dans les conditions physiologiques normales, l'activité des facteurs de coagulation est régulée par des protéines telles que l'antithrombine III (AT III) et le cofacteur II de l'héparine (HC II), qui sont également présentes dans le plasma. L'AT III exerce une activité inhibitrice sur un certain nombre de facteurs de coagulation et notamment sur les facteurs Xa et IIa.

L'inhibition du facteur Xa ou du facteur IIa constitue donc un moyen privilégié pour obtenir une activité anticoagulante et antithrombotique puisque ces deux facteurs interviennent dans les deux dernières étapes de la coagulation, lesquelles sont indépendantes du stimulus déclencheur.

Le pentasaccharide de formule:

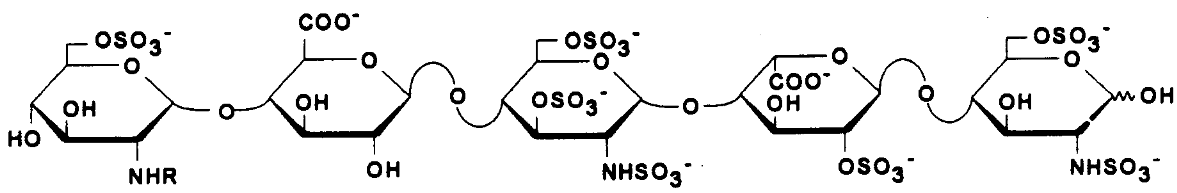

(1) R = $-COCH_3$
(2) R = $-SO_3^-$

représente la séquence minimale de l'héparine, requise pour la liaison à l'AT III. Ce composé (R = $-SO_3^-$) a été obtenu il y a environ une dizaine d'années par synthèse chimique totale (P. Sinaÿ et al, Carbohydrate Research (1984), 132 C5).

Depuis, un certain nombre d'oligosaccharides de synthèse, obtenus par synthèse chimique totale et ayant des activités antithrombotiques et anticoagulantes, ont été décrits dans la littérature.

Le brevet EP-0 084 999 décrit des dérivés constitués d'unités monosaccharidiques acides uroniques (glucuronique ou iduronique) et glucosamine et possédant des propriétés antithrombotiques intéressantes. En dehors des substituants groupes hydroxy, ces composés contiennent des groupes N-sulfate, des groupes N-acétyle et dans certains cas, les groupes anomériques hydroxy sont remplacés par des groupes méthoxy.

La demande EP-0 165 134 décrit aussi des oligosaccharides de synthèse ayant des activités antithrombotiques. Ces composés sont constitués d'unités monosaccharidiques acides uroniques et glucosamine et contiennent des groupes O-sulfate ou O-phosphate. Des dérivés d'acides uroniques et de glucosamine comportant en position 3 de l'unité glucosamine un groupe O-sulfate, sont aussi décrits dans la demande EP-0 301 618. Ces composés possèdent des propriétés antithrombotiques et anticoagulantes élevées. La demande de brevet EP-0 454 220 décrit des dérivés d'acides uroniques et de glucose possédant comme substituants des groupes O-alkyle ou O-sulfate. Ces derniers composés sont aussi doués de propriétés antithrombotiques et anticoagulantes.

Des dérivés glycosaminoglycanoïdes sulfatés dans lesquels les groupes fonctionnels N-sulfate, N-acétate ou hydroxy ont été remplacés par des groupes alcoxy, aryloxy, aralkyloxy ou O-sulfates sont aussi décrits dans la demande EP-0 529 715. Ces composés ont des propriétés antithrombotiques intéressantes. Ces derniers sont aussi des inhibiteurs de la prolifération des cellules musculaires lisses.

Des oligosaccharides, et notamment des pentasaccharides, analogues de la séquence minimale de l'héparine requise pour la liaison à l'AT-III, sont décrits dans Angew. Chem. Int. Ed. Engl. (1993), 32, (3), pp. 434-436. Ces composés contiennent des unités acides glucuroniques ou glucose dont les fonctions hydroxy ont été remplacées par des groupes O-sulfate ou O-méthyle.

On a maintenant trouvé de manière surprenante qu'en remplaçant un ou plusieurs radicaux hydroxy, ou des groupes O-alkyle, ou O-sulfate en position 3 par des atomes d'hydrogène, d'une ou plusieurs unités saccharidiques, on obtient des oligosaccharides doués de propriétés biologiques intéressantes. En effet, les composés de la présente invention se distinguent des autres héparinoïdes de synthèse décrits dans la littérature, par leurs structures originales et par leurs propriétés biologiques puissantes et inattendues. Les composés de l'invention sont des 3-désoxy oligosaccharides possédant une très grande activité anti-facteur Xa, et une grande affinité pour l'AT III. Par ailleurs, les composés de l'invention sont bien absorbés par le tractus digestif. Ce sont donc des produits qui peuvent être administrés par voie orale.

La présente invention a plus particulièrement pour objet, les composés de formule I:

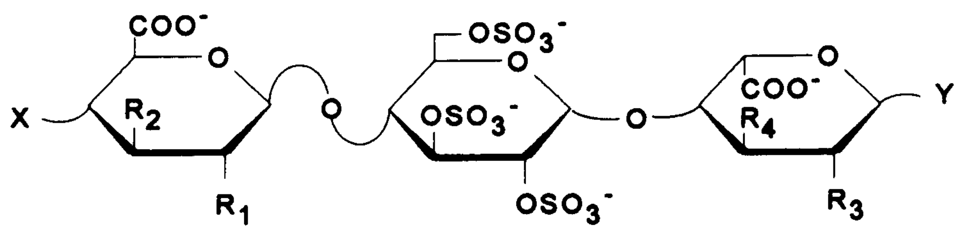

(I)

dans laquelle,

- X représente un radical $-OSO_3^-$, un radical de formule A,

$$R—O \quad (A)$$

un radical de formule B,

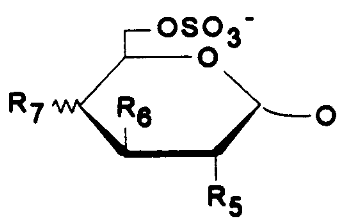

(B)

ou un radical de formule C,

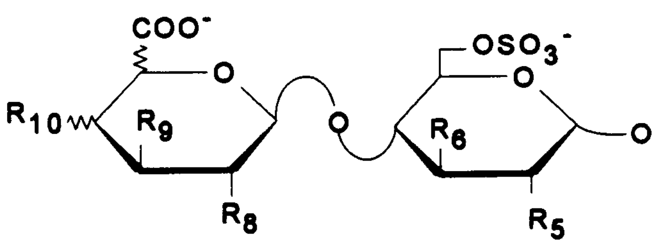

(C)

- Y représente un radical de formule D,

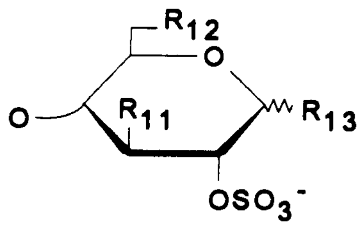

(D)

- R représente un radical alkyle, linéaire ou ramifié de 1 à 6 atomes de carbone,
- $R_1$, $R_3$, $R_5$, $R_7$, $R_8$, $R_{10}$, $R_{12}$ et $R_{13}$ identiques ou différents, représentent chacun un radical hydroxy, un radical alcoxy linéaire ou ramifié de 1 à 6 atomes de carbone, ou un radical $-OSO_3^-$,
- $R_2$, $R_4$, $R_6$, $R_9$ et $R_{11}$, identiques ou différents représentent chacun un atome d'hydrogène, un radical hydroxy, un radical alcoxy linéaire ou ramifié de 1 à 6 atomes de carbone, ou un radical $-OSO_3^-$,

à condition toutefois qu'ou moins un des substituants présents $R_2$ ou $R_4$ ou $R_6$ ou $R_9$ ou $R_{11}$, représente un atome d'hydrogène,

sous forme de sels pharmaceutiquement acceptables et les acides correspondants.

Par la suite, le terme "unité monosaccharidique", sera utilisé pour désigner l'unité osidique :

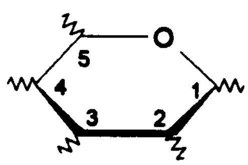

indépendamment des substituants qui seront liés à cette unité, en positions 2, 3 ou 5.

Concernant les radicaux B, C et D, la liaison ≷ signifie que dans certains cas, la configuration du carbone portant le substituant attaché par cette liaison, peut-être R et dans d'autres cas, S.

Les composés de la présente invention sont des dérivés de 3-désoxysaccharides.

Il faut donc qu'au moins une des unités monosaccharidiques à partir de laquelle les composés de l'invention sont formés, corresponde à la structure d'un 3-désoxymonosaccharide.

Il en résulte que dans la formule I, au moins un des substituants $R_2$ ou $R_4$ ou $R_6$ ou $R_9$ ou $R_{11}$ représente un atome d'hydrogène.

Les composés de formule I dans laquelle $R_2$ représente un atome d'hydrogène sont des produits préférés de l'invention.

Les composés préférés de la présente invention sont aussi les composés de formule I, dans laquelle, X représente un radical de formule (B) ou un radical de formule (C).

Ces composés répondent aux formules Ia et Ib suivantes:

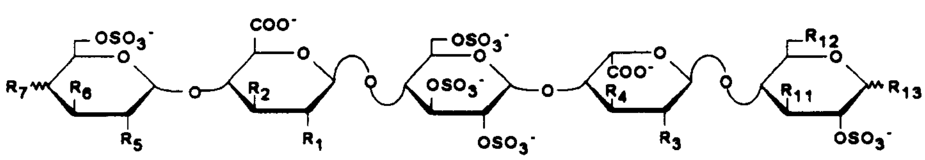

(Ia)

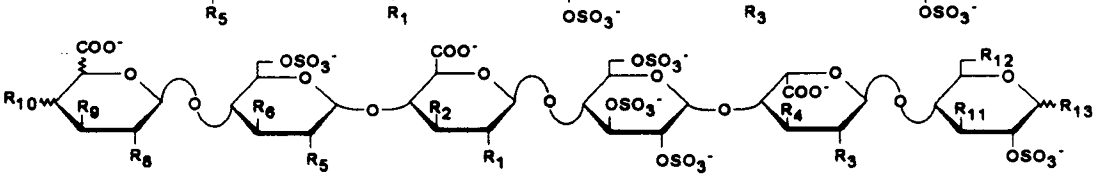

(Ib)

dans lesquelles $R_1$, $R_2$, $R_3$, $R_4$, $R_5$, $R_6$, $R_7$, $R_8$, $R_9$, $R_{10}$, $R_{11}$, $R_{12}$, et $R_{13}$, ont la même signification que pour la formule I.

On préfère plus particulièrement les composés de formule Ia.

Les composés de formule I et plus particulièrement, ceux des formules Ia et Ib, dans lesquelles:

- $R_1$, $R_3$, $R_5$, $R_7$, $R_8$, $R_{10}$, et $R_{13}$ identiques ou différents, représentent chacun un radical alcoxy linéaire ou ramifié de 1 à 6 atomes de carbone, ou un radical $-OSO_3^-$,
- $R_2$, $R_4$, $R_6$ et $R_9$, identiques ou différents représentent chacun un atome d'hydrogène ou un radical alcoxy linéaire ou ramifié de 1 à 6 atomes de carbone,
- $R_{11}$ représente un atome d'hydrogène, un radical alcoxy linéaire ou ramifié de 1 à 6 atomes de carbone ou un radical $-OSO_3^-$,

  à condition toutefois qu'au moins $R_2$ ou $R_4$ ou $R_6$ ou $R_9$ ou $R_{11}$ représente un atome d'hydrogène,

  et,
- $R_{12}$ représente un radical hydroxy ou un radical $-OSO_3^-$, sont des composés préférés de l'invention.

On préfère plus particulièrement les composés de formule I et notamment, ceux des formules Ia et Ib dans lesquelles:

- $R_2$ et $R_6$ représentent un atome d'hydrogène,
- $R_3$, $R_{11}$ et $R_{12}$ représentent un radical $-OSO_3^-$,

  et,
- $R_{13}$ représente un radical alcoxy linéaire ou ramifié de 1 à 6 atomes de carbone.

On préfère aussi plus particulièrement les composés de formule I et notamment, ceux des formules Ia et Ib, dans lesquelles le radical alcoxy est un radical méthoxy.

La présente invention a également pour objet un procédé de préparation des composés de formule I,

caractérisé en ce que l'on fait réagir un composé de formule II :

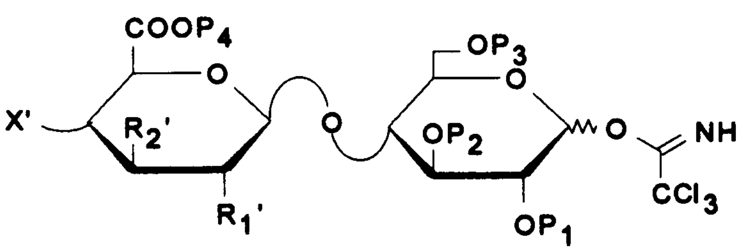

(II)

dans laquelle,

- X' représente un radical chloroacétoxy, un radical lévulinyloxy, un radical de formule A, un radical de formule $B_1$,

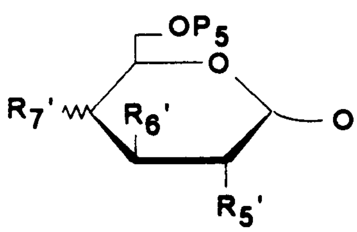

($B_1$)

ou un radical de formule $C_1$,

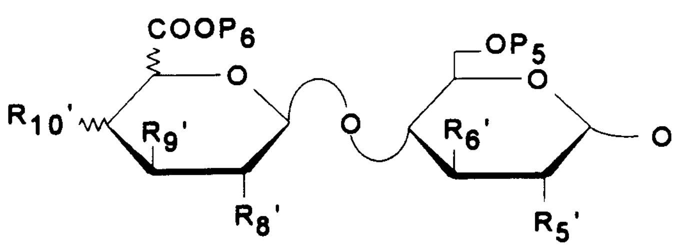

($C_1$)

- $P_1$, $P_2$, $P_3$ et $P_5$, identiques ou différents, représentent chacun un groupe protecteur tel qu'un radical acyle acyclique de 1 à 6 atomes de carbone, de préférence un radical acétyle, un radical acyle aromatique, de préférence un radical benzoyle, un radical alc-2-ényle de 2 à 7 atomes de carbone, de préférence un radical allyle, ou un radical benzyle,
- $P_4$, $P_6$, identiques ou différents représentent chacun un groupe protecteur tel qu'un radical alkyle de 1 à 6 atomes de carbone de préférence un radical méthyle ou, un radical benzyle,
- $R_1'$, $R_5'$, $R_8'$, identiques ou différents, représentent chacun un radical alcoxy linéaire ou ramifié de 1 à 6 atomes de carbone, un radical acyloxy acyclique de 1 à 6 atomes de carbone, de préférence un radical acétoxy, un radical acyloxy aromatique, de préférence un radical benzoyloxy, ou un radical alc-2-èn yloxy de 2 à 7 atomes de carbone, de préférence un radical allyloxy,
- $R_7'$, $R_{10}'$, identiques ou différents, ont les significations données pour $R_1'$ ou ils représentent un radical chloroacétoxy ou un radical lévulinyloxy,
  et,
- $R_2'$, $R_6'$ et $R_9'$, identiques ou différents, représentent chacun un atome d'hydrogène, ou ont les significations données pour $R_1'$,

avec un composé de formule III:

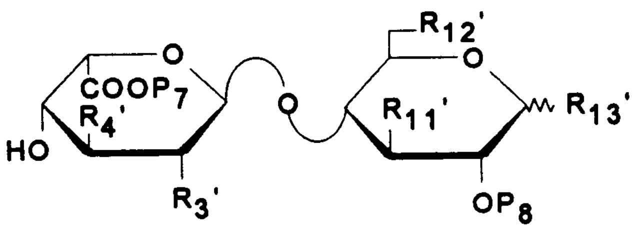
(III)

dans laquelle,

- P7 a les significations données pour $P_4$ dans la formule II,
- $P_8$ a les significations données pour $P_1$ dans la formule II,
- $R_4'$ et $R_{11}'$ identiques ou différents, ont les mêmes significations que $R_2'$ dans la formule II,
  et,
- $R_3'$, $R_{12}'$ et $R_{13}'$ identiques ou différents ont les significations données pour $R_1'$ dans la formule II,

pour préparer les composés de formule IV:

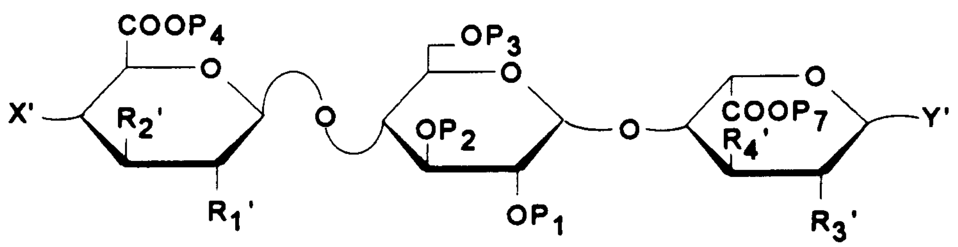
(IV)

dans laquelle,

- X', $P_1$, $P_2$, $P_3$, $P_4$, $R_1'$ et $R_2'$ ont la même signification que pour la formule II,
- $P_7$, $R_3'$ et $R_4'$ ont la même signification que pour la formule III, et,
- Y' représente un radical de formule $D_1$:

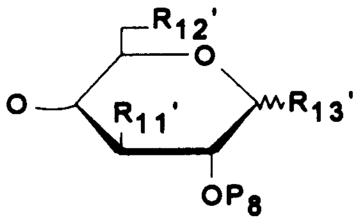

(D$_1$)

dans laquelle,

- $P_8$, $R_{11}$', $R_{12}$' et $R_{13}$' ont la même signification que pour la formule III,

que l'on soumet ensuite soit à une hydrogénation catalytique puis à une saponification et à une sulfatation, soit d'abord à une saponification, puis à une sulfatation et ensuite à une hydrogénation catalytique, soit d'abord à une hydrogénation catalytique, puis à une sulfatation et ensuite à une saponification pour obtenir les composés de formule I.

Le procédé décrit ci-dessus est le procédé préféré de l'invention. Toutefois, les composés de formule I peuvent être préparés par d'autres méthodes connues de la chimie des sucres, et notamment, en faisant réagir un monosaccharide comportant des groupes protecteurs tels que décrits par T.W. Green, dans Protective Groups in Organic Synthesis (Wiley, N.Y. 1981) sur les radicaux hydroxy et éventuellement sur les radicaux carboxy s'il en dispose, avec un autre monosaccharide protégé, pour former un disaccharide sur lequel ensuite, on fait réagir un autre monosaccharide protégé, pour former un trisaccharide protégé à partir duquel, on peut obtenir un tetrasaccharide protégé puis un pentasaccharide protégé et ensuite, un hexasaccharide protégé (approche "pas à pas").

Les oligosaccharides protégés (tetra, penta et hexasaccharide) sont ensuite déprotégés et éventuellement sulfatés ou, d'abord partiellement déprotégés puis sulfatés et ensuite déprotégés, pour obtenir des composés de formule I.

De tels procédés sont connus dans la chimie des glucides, et particulièrement décrits par G. Jaurand et *al* dans Bioorganic and Medicinal Chemistry Letters (1992), 2 (n°9), pp. 897-900, par J. Basten et *al*, dans Bioorganic and Medicinal Chemistry Letters (1992), 2 (n°9), pp. 901-904, par J. Basten et *al*, dans Bioorganic and Medicinal Chemistry Letters (1992), 2 (n°9), pp. 905-910 et par M. Petitou et C.A.A. van Boeckel dans "Chemical Synthesis of heparin fragments and analogues" pp. 203-210 - Progress in the Chemistry of Organic Natural Products, Ed. Springer Verlag Wien - N.Y. (1992).

Les composés de formule II, lorsque X' représente un radical de formule $B_1$, peuvent être préparés en faisant réagir un monosaccharide activé sur son carbone anomère, comme par exemple un composé de formule V:

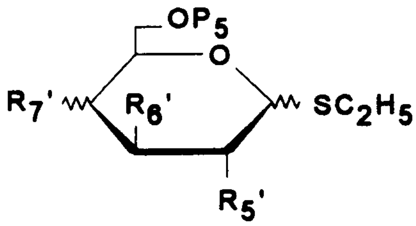

(V)

dans laquelle,

- $P_5$, $R_5$', $R_6$' et $R_7$', ont la même signification que pour la formule $B_1$,

avec un composé de formule VI :

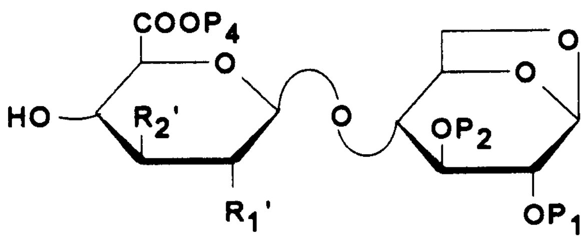

(VI)

dans laquelle,

- $R_1$', $R_2$', $P_1$, $P_2$ et $P_4$ ont la même signification que pour la formule II,

pour obtenir ainsi un composé de formule VII :

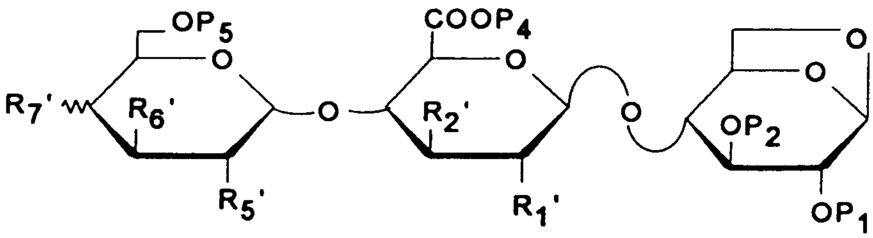

(VII)

dans laquelle,

- $P_5$, $R_5$', $R_6$' et $R_7$', ont la même signification que pour la formule V,
  et,
- $R_1$', $R_2$', $P_1$, $P_2$ et $P_4$ ont la signification donnée pour la formule II,

selon le procédé décrit par T. Peters et *al* dans Can. J. Chem., (1989), 67, pp. 491-496, et par G.H. Veeneman et J.H. van Boom dans Tetraltedron Letters (1990), 31, pp. 275-278.

En traitant ce composé selon des procédés connus (R. Schmidt, Angew. Chem. Int. Ed, Engl. (1986), 25, (3), pp. 212-235), et notamment par acétolyse, traitement par la benzylamine puis par le thichloroacétonitrile, on obtient les composés de formule II, dans laquelle X' représente un radical de formule $B_1$.

La préparation de certains composés de formule VII, et notamment des composés pour lesquels $R'_2$ et $R'_6$ ont les significations données pour $R'_1$ et ne représentent pas un atome d'hydrogène, est décrite par J. Basten et *al*, (Bioorganic and Medicinal Chemistry Letters (1992), 2 (n°9), pp. 905-910).

Les composés de formule VII peuvent être aussi préparés en faisant réagir les composés VI avec d'autres monosaccharides activés par exemple avec les composés de formule V' :

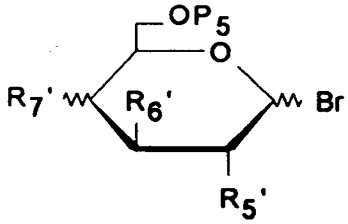

(V')

dans laquelle,

- $P_5$, $R_5$', $R_6$' et $R_7$', ont la même signification que pour la formule V,

Les composés de formule V' pour lesquels $R_6$' a les significations données pour $R_1$', et ne représente pas un atome d'hydrogène, sont des composés connus et décrits par M. Petitou et C.A.A. van Boeckel dans "Chemical Synthesis of

heparin fragments and analogues" pp. 203-210 - Progress in the Chemistry of Organic Natural Products, Ed. Springer Verlag Wien - N.Y. (1992).

Des composés de formule V dans laquelle $R_6$' représente un atome d'hydrogène peuvent être obtenus à partir d'un composé de formule VIII :

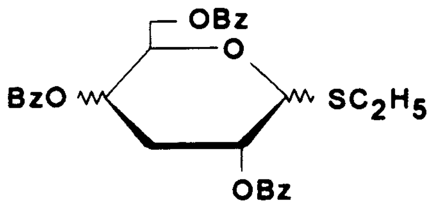

(VIII)

dans laquelle Bz représente un radical benzoyle.

Le composé de formule VIII est préparé à partir du 3-désoxy-β-D-*ribo*-hexopyranose (préparé selon la méthode de T.V. Rajanbabu décrite dans J. Org. Chem., (1988), 53, pp. 4522-4530), lequel est soumis à l'action de chlorure de benzoyle dans un solvant organique basique. Le composé ainsi obtenu est soumis à l'action d'éthanethiol, pour obtenir les composés de formule VIII.

Les composés de formule VI, quand $R_2$' représente un atome d'hydrogène, peuvent aussi être obtenus à partir du composé de formule VIII, que l'on fait réagir avec du 1,6:2,3-di-anhydro-β-D-mannopyranose,

pour obtenir un composé de formule IX :

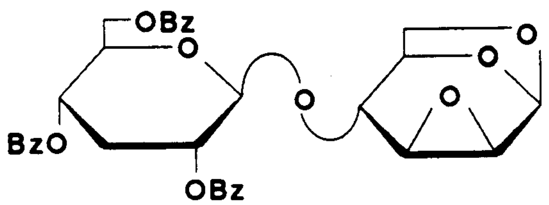

(IX)

dans laquelle Bz représente un radical benzoyle.

Ce composé est ensuite soumis à l'action d'une base forte pour obtenir le 1,6:2,3-di-anhydro-4-O-(3-désoxy-β-D-*ribo*-hexopyranosyl)-β-D-mannopyranose, composé de formule X:

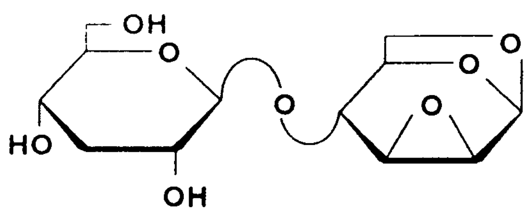

(X)

A partir du composé de formule X, et en utilisant des méthodes classiques [G. Jaurand et *al*, Bioorganic and Medicinal Chemistry Letters (1992), 2 (n°9), pp. 897-900 ; J. Basten et *al*, Bioorganic and Medicinal Chemistry Letters (1992), 2 (n°9), pp. 901-904 ; J Basten et *al*, Bioorganic and Medicinal Chemistry Letters (1992), 2 (n°9), pp. 905-910 ; M. Petitou et C.A.A. van Boeckel "Chemical Synthesis of heparin fragments and analogues" pp. 203-210 - Progress in the Chemistry of Organic Natural Products, Ed. Springer Verlag Wien - N.Y. (1992)], on obtient les composés de formule XI :

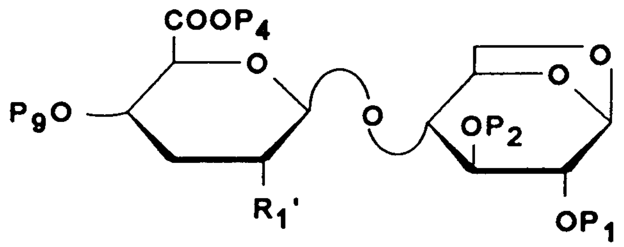

(XI)

dans laquelle,

- $R_1$', $P_1$, $P_2$ et $P_4$ ont la même signification que pour la formule VII, et,
- $P_9$ représente un radical lévulinyle ou un radical chloroacétyle.

Le composé de formule XI est ensuite soumis à l'action de l'hydrazine pour obtenir les composés de formule VI dans laquelle $R_2$' représente un atome d'hydrogène.

Les composés de formule IX, X et XI sont des produits nouveaux, et font aussi partie de l'invention.

Les composés de formule VI pour lesquels $R_2$' a les significations données pour $R_1$' et qui ne représente pas un atome d'hydrogène, peuvent être obtenus de manière analogue en utilisant comme produits de départ, des dérivés protégés de glucose, activés au niveau de leur carbone anomère, au lieu des composés de formules VIII. La préparation de tels composés est décrite par M. Petitou et C.A.A. van Boeckel dans "Chemical Synthesis of heparin fragments and analogues" pp. 203-210 - Progress in the Chemistry of Organic Natural Products, Ed. Springer Verlag Wien - N.Y. (1992). Les mêmes auteurs décrivent aussi la préparation d'analogue des composés de formule IX contenant en position 3', un radical hydroxyle protégé.

Les composés de formule II, lorsque X' représente un radical de formule $C_{1a}$ :

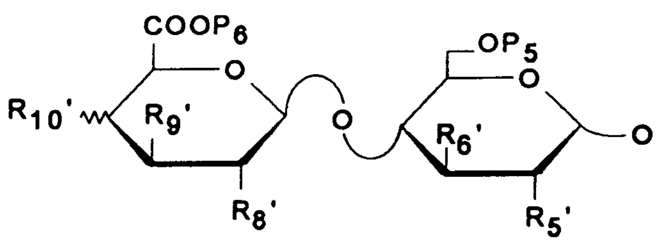

($C_{1a}$)

dans laquelle,

- $R_5$', $R_6$', $R_8$', $R_9$', $R_{10}$', $P_5$ et $P_6$, ont la même signification que pour la formule $C_1$,

peuvent être obtenus en faisant réagir un composé de formule VI avec un composé de formule XIIa :

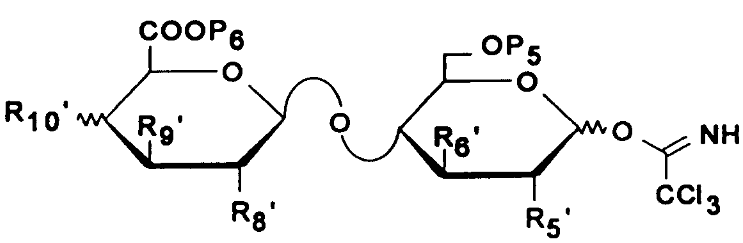

(XIIa)

dans laquelle $R_5$', $R_6$', $P_8$', $R_9$', $R_{10}$', $P_5$ et $P_6$, ont la même signification que pour la formule $C_1$,
pour obtenir un composé de formule XIIIa :

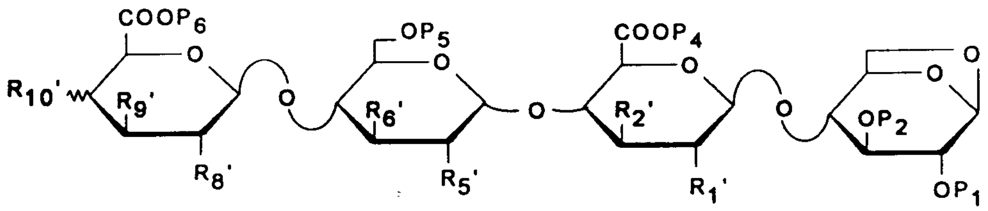 (XIIIa)

dans laquelle,

- $R_5'$, $R_6'$, $R_8'$, $R_9'$, $R_{10}'$, $P_5$ et $P_6$, ont la même signification que pour la formule $C_1$ et,
- $R_1'$, $R_2'$, $P_1$, $P_2$ et $P_4$, ont les significations données pour la formule II.

Les composés de formule XIIIa sont ensuite traités comme indiqué pour le composé VII, pour obtenir les composés de formule II, dans laquelle X' représente un radical de formule $C_{1a}$.

Les composés de formule XIIa quand $R_9'$ représente un atome d'hydrogène, sont obtenus à partir des composés de formule XIV :

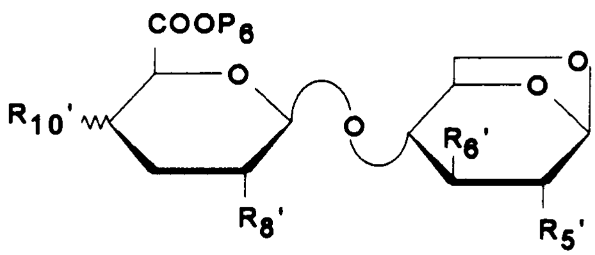 (XIV)

dans laquelle,

- $R_5'$, $R_6'$, $R_8'$, $R_{10}'$ et $P_6$, ont la même signification que pour la formule $C_1$,

lequel est soumis à une acétolyse, à une déprotection puis à l'action de trichloroacétonitrile. Les composés de formule XIV peuvent être obtenus à partir des composés de formule X, selon le procédé décrit pour les composés de formule XI.

Les composés de formule XIIa quand $R_9'$ a les significations données pour $R_1'$ et ne représente pas un atome d'hydrogène, sont préparés à partir des dérivés du 1,6:2,3-di-anhydro-4-O-β-D-glucopyranosyl)-β-D-mannopyranose en appliquant le procédé décrit ci-dessus. Les dérivés du 1,6:2,3-di-anhydro-4-O-(β-D-glucopyranosyl)-β-D-mannopyranose sont des produits connus et décrits par J. Basten et *al* dans Bioorganic and Medicinal Chemistry Letters (1992), 2 (n°9), pp. 905-910.

Les composés de formule II, lorsque X' représente un radical de formule $C_{1b}$ :

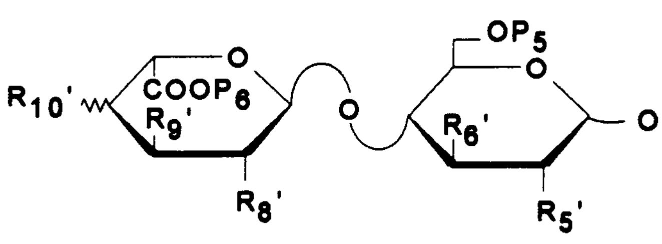 ($C_{1b}$)

dans laquelle,

- $R_5'$, $R_6'$, $R_8'$, $R_9'$, $R_{10}'$, $P_5$ et $P_6$, ont la même signification que pour la formule $C_1$,

peuvent être obtenus en faisant réagir un composé de formule XIIb :

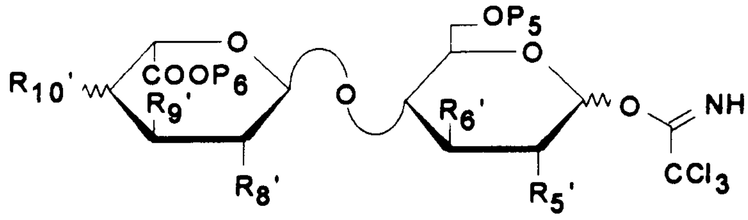

(XIIb)

dans laquelle,

- $R_5'$, $R_6'$, $R_8'$, $R_8'$, $R_{10}'$, $P_5$ et $P_6$, ont les significations données pour la formule $C_1$,

avec un composé de formule VI, pour obtenir les composés de formule XIIIb :

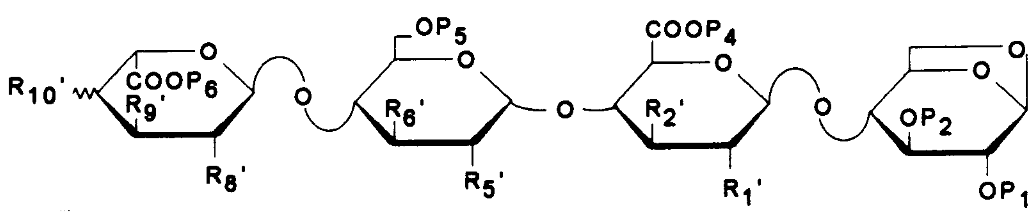

(XIIIb)

dans laquelle,

- $R_1'$, $R_2'$, $R_5'$, $R_6'$, $R_8'$, $R_9'$, $R_{10}'$, $P_1$, $P_2$, $P_4$, $P_5$ et $P_6$ ont les mêmes significations que pour la formule XIIIa.

Les composés de formule XIIIb sont ensuite traités comme indiqué pour les composés de formule XIIIa pour obtenir les composés de formule II, dans laquelle X' représente un radical de formule $C_{1b}$.

Les composés de formule XIIb, quand $R_9'$ représente un atome d'hydrogène, peuvent être obtenus à partir des composés de formule X. Ce composé est transformé en un composé de formule XV :

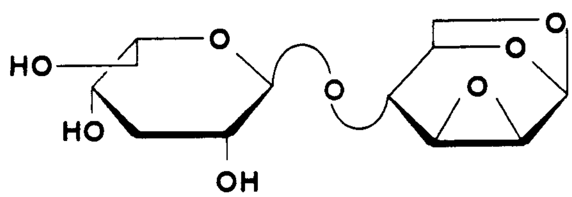

(XV)

selon un procédé équivalent à celui décrit par Ichikawa et *al,* dans Carbohydrate Research, (1988), 172, pp. 37-64.

A partir de ce composé, et en utilisant des méthodes connues décrites par G. Jaurand et *al* dans Bioorganic and Medicinal Chemistry Letters (1992), 2 (n°9), pp. 897-900, par J. Basten et *al* dans Bioorganic and Medicinal Chemistry Letters (1992), 2 (n°9), pp. 901-904, par J. Basten et *al* dans Bioorganic and Medicinal Chemistry Letters (1992), 2 (n°9), pp. 905-910 et par M. Petitou et C.A.A. van Boeckel "Chemical Synthesis of heparin fragments and analogues" pp. 203-210 - Progress in the Chemistry of Organic Natural Products, Ed. Springer Verlag Wien - N.Y. (1992), on obtient les composés de formule XVI :

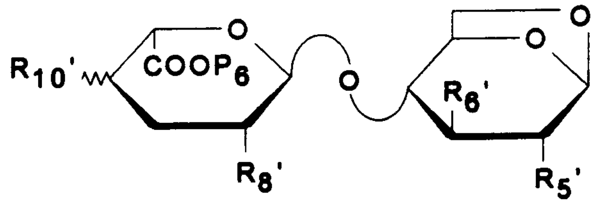

(XVI)

dans laquelle,

- $R_5'$, $R_6'$, $R_8'$, $R_{10}'$ et $P_6$, ont la même signification que pour la formule XIIb.

Ces composés sont ensuite soumis à une acétolyse, puis traités par la benzylamine et le trichloroacétonitrile, pour obtenir les composés de formule XIIb.

Les composés de formule XIIb pour lesquels $R_9'$ ne représente pas un atome d'hydrogène mais a les significations données pour $R_1'$, peuvent être obtenus de la même manière.

Les différents intermédiaires permettant d'obtenir les composés de formule XIIb, dans laquelle, $R_5'$, $R_6'$, $R_8'$, $R_{10}'$, $P_5$ et $P_6$, ont la même signification que pour la formule $C_1$, et $R_9'$ a les significations données pour $R_1'$ et ne représente pas un atome d'hydrogène, sont des produits connus et leur préparation est décrite par G. Jaurand et *al* dans Bioorganic and Medicinal Chemistry Letters (1992), 2 (n°9), pp. 897-900.

Les composés de formule II, lorsque X' représente un radical de formule A et $R_2'$ représente un atome d'hydrogène, peuvent être obtenus à partir du composé de formule X, lequel est transformé en un composé de formule XVII :

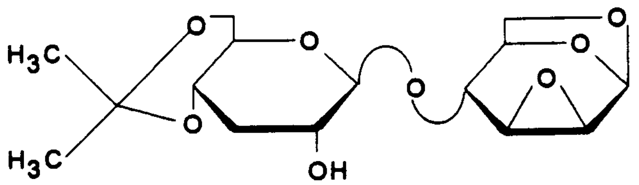

(XVII)

Le composé de formule XVII est par la suite, soit traité par le benzylate de sodium, puis acylé, soit traité par un halogénure d'aralkyle de préférence le bromure de benzyle, un halogénure d'alc-2-én yle de préférence le bromure d'allyle puis traité par le benzylate de sodium et ensuite acylé, pour obtenir les composés de formule XVIII :

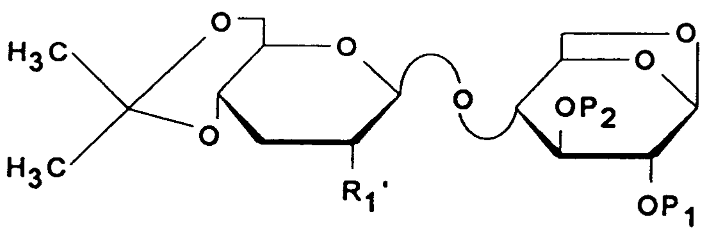

(XVIII)

dans laquelle $R_1'$, $P_1$ et $P_2$ ont la même signification que pour la formule II.

Les composés de formule XVIII sont traités ensuite en milieu acide pour obtenir les composés de formule XIX :

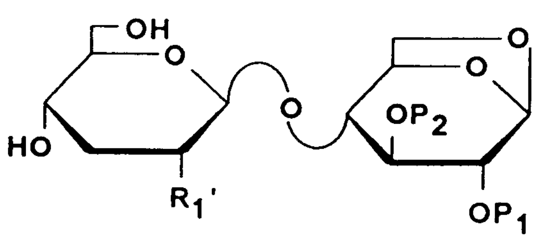

(XIX)

dans laquelle $R_1'$, $P_1$ et $P_2$, ont les significations données pour la formule II.

Les composés de formule XIX sont ensuite soumis à une silylation sélective en position 6', traités par l'anhydride lévulinique, oxydés selon les conditions de Jones, et estérifiés à l'aide d'un halogénure d'alkyle, soumis à une élimination du radical lévulinyle, et alkylés en milieu acide ou neutre, pour obtenir les composés de formule II dans laquelle X' représente un radical de formule A, et $R_2'$ représente un atome d'hydrogène.

De la même manière, peuvent être obtenus les composés de formule II, lorsque X' représente un radical de formule A et $R_2'$ a les significations données pour $R_1'$. Les intermédiaires nécessaires pour la préparation de ces composés sont décrits dans la littérature et notamment par J. Basten et *al*, dans Bioorganic and Medicinal Chemistry Letters (1992), 2 (n°9), pp. 905-910, et par C.A.A. van Boeckel et al dans J. Carbohydr. Chem. (1985), 4, p. 293.

Les composés de formule III, quand $R_4'$ représente un atome d'hydrogène, peuvent être préparés à partir des com-

posés de formule XX :

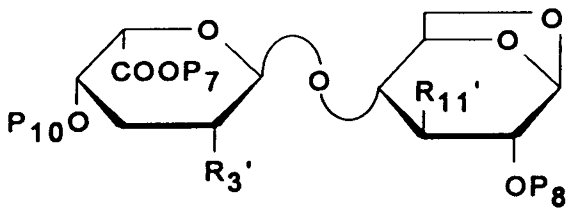

(XX)

dans laquelle,

- $R_3'$, $R_{11}'$, $P_7$ et $P_8$ ont les significations données pour la formule III, et
- $P_{10}$ représente un radical lévulinyle ou un radical chloroacétyle.

Les composés de formule XX sont soumis à une acétolyse, puis traités par la benzylamine, mis en réaction avec le réactif de Vilsmeïer, traités par un alcool en présence du carbonate d'argent et soumis ensuite à l'action de l'hydrazine, pour obtenir les composés de formule III attendus ($R_4'$ = H).

Les composés de formule III quand $R_4'$ à les mêmes significations que $R_1'$, sont des composés connus. La préparation de tels composés est décrite dans la littérature par J. Basten et *al* dans Bioorganic and Medicinal Chemistry Letters (1992), 2 (n°9), pp. 905-910.

Le procédé décrit ci-dessus permet d'obtenir les composés de l'invention sous forme de sels. Pour obtenir les acides correspondants, les composés de l'invention sous forme de sels, sont mis en contact avec une résine échangeuse de cations sous forme acide.

Les composés de l'invention sous forme d'acides peuvent être ensuite neutralisés par une base pour obtenir un sel souhaité.

Pour la préparation des sels des composés de formule I, on peut utiliser toute base minérale ou organique, donnant avec les composés de formule I, des sels pharmaceutiquement acceptables.

On utilise de manière préférentielle l'hydroxyde de sodium, de potassium, de calcium ou de magnésium. Les sels de sodium et de calcium des composés de formule I, sont les sels préférés.

Les composés de formule I, objet de la présente invention, ont des propriétés pharmacologiques et biochimiques intéressantes. Ils possèdent plus particulièrement une grande activité anti-facteur Xa, et une grande affinité pour l'AT III.

Comme cela a été mentionné précédemment, le facteur Xa active dans la cascade de coagulation la prothrombine en thrombine, qui protéolyse le fibrinogène soluble avec libération de la fibrine insoluble, constituant principal du caillot sanguin. L'inhibition du facteur Xa constitue donc un moyen privilégié pour obtenir une activité anticoagulante et antithrombotique.

L'activité anti-facteur Xa (anti-Xa) des produits de l'invention a été évaluée à pH 8,4, selon la méthode décrite par Teien A.N. et Lie M., dans Thrombosis Research (1977), 10, pp. 399-410, et il a été démontré que les produits de l'invention possèdent une activité anti-Xa égale ou supérieure à celle des héparinoïdes de synthèse déjà connus.

L'affinité des composés de formule I pour l'AT III, a été déterminée par spectrofluorométrie, dans les conditions décrites par D. Atha et *al* dans Biochemistry (1987), 26, pp. 6454-6461. Les résultats des essais ont démontré que-les composés de l'invention possèdent une très grande affinité pour l'AT III.

Par ailleurs, l'activité antithrombotique globale des produits de formule I a été évaluée chez le rat, par un modèle de stase veineuse et induction par la thromboplastine, selon la méthode décrite par J. Reyers et *al* dans Thrombosis Research (1980), 18, pp. 669-674. La $DE_{50}$ des composés de l'invention est au moins du même ordre ou inférieure à celle des autres héparinoïdes de synthèse déjà connus. Les composés de l'invention présentent donc une spécificité d'action et une activité anticoagulante et antithrombotique particulièrement intéressantes.

Les résultats obtenus lors de différentes études pharmacocinétiques effectuées avec les produits de l'invention, ont démontré qu'ils sont très bien absorbés et que leur demi-vie est grande. Cela permet d'envisager lors de leurs utilisations en thérapeutique, la possibilité d'une administration unique journalière.

Ces études ont aussi démontré que les produits de formule I, objet de la présente invention, sont absorbés par le tractus digestif, sans que les quantités administrées ne soient prohibitives pour une utilisation en thérapeutique humaine. Les composés de l'invention sont donc utiles pour la préparation des compositions pharmaceutiques, administrables aussi bien par voie parentérale, que par voie orale.

Les composés de formule I sont très peu toxiques ; leur toxicité est parfaitement compatible avec leur utilisation comme médicaments.

Les composés de l'invention peuvent aussi trouver leurs applications dans le traitement de la prolifération des cellules musculaires lisses, puisqu'il a été démontré qu'ils exercent un effet inhibiteur, sensiblement supérieur à celui de l'héparine, sur la croissance des cellules musculaires lisses.

Les composés de l'invention sont aussi actifs sur l'angiogénèse et utiles pour le traitement de certaines infections dues aux rétrovirus.

Par ailleurs, les composés de l'invention exercent aussi une action protectrice et régénératrice sur les fibres nerveuses.

Les composés de l'invention sont très stables et sont donc ainsi particulièrement appropriés pour constituer le principe actif de médicaments.

L'invention s'étend aussi aux compositions pharmaceutiques contenant comme principe actif, un composé de formule I ou l'un de ses sels pharmaceutiquement acceptables, éventuellement en association avec un ou plusieurs excipients inertes et appropriés.

Les compositions pharmaceutiques ainsi obtenues sont présentées avantageusement sous des formes diverses, telles que par exemple, des solutions injectables ou buvables, dragées, comprimés ou gélules. Les solutions injectables sont les formes pharmaceutiques préférées.

Les compositions pharmaceutiques contenant comme principe actif au moins un composé de formule I ou l'un de ses sels, sont notamment utiles pour le traitement à titre préventif ou curatif, des troubles de la paroi vasculaire, tels que l'athérosclérose et l'artériosclérose, les états d'hypercoagulabilité observés par exemple à la suite d'opérations chirurgicales, de développements tumoraux ou de dérèglements de la coagulation, induits par des activateurs bactériens viraux, ou enzymatiques.

La posologie peut largement varier en fonction de l'âge, du poids et de l'état de santé du patient, de la nature et de la sévérité de l'affection, ainsi que de la voie d'administration. Cette posologie comprend l'administration d'une ou plusieurs doses d'environ 0,5 mg à 1000 mg par jour, de préférence d'environ 1 à 100 mg par jour par exemple de l'ordre de 20 mg par jour, par voie intramusculaire ou par voie sous cutanée, en administrations discontinues ou à intervalles réguliers, ou d'une dose journalière de l'ordre de 200 mg à 1000 mg par jour par voie orale.

Ces doses peuvent naturellement être ajustées pour chaque patient, en fonction des résultats observés et des analyses de sang préalablement effectuées. L'administration par voie sous-cutanée est la voie préférée.

L'invention est illustrée par les exemples ci-après.

## PREPARATIONS

### PREPARATION I

**Ethyl 2,4,6-tri-O-benzoyl-3-désoxy-1-thio-D-*ribo*-hexopyranoside (Composé de formule VIII)**

STADE A

1,2,4,6-tétra-O-benzoyl-3-désoxy-β-D-*ribo*-hexopyranose

Chauffer à 60°C pendant 4 heures, 66 mmole de 3-désoxy1,2:5,6-di-O-isopropylidène D-*ribo*-hexofuranose (T.V. Rajanbabu, J. Org. Chem. (1988), 53, pp. 4522-4530), en solution dans un mélange d'eau et d'éthanol et en présence d'une résine acide Dowex pour obtenir du 3-désoxy-D-*ribo*-hexopyranose Evaporer à sec, puis, sécher en évaporant en présence de pyridine.

Dissoudre le sirop ainsi obtenu dans 150 ml de pyridine et ajouter 356 mmole de chlorure de benzoyle. Laisser sous agitation à température ambiante pendant 3 heures. Evaporer à sec, diluer dans du dichlorométhane, laver à l'eau et cristalliser dans l'acétate d'éthyle pour obtenir 7,66 g de 1,2,4,6-tétra-O-benzoyl-3-désoxy-β-D-*ribo*-hexopyranose.
Rendement : 30 %
Point de fusion : 164°C
$[\alpha]_D^{20}$ = = + 1° (C = 1,33 dans $CH_2Cl_2$)

STADE B

Dissoudre dans du toluène anhydre et sous atmosphère d'argon à 20°C, 4,51 mmole du composé obtenu au stade précédent, puis ajouter 9,03 mmole d'éthanethiol.

Ajouter 4,51 mmole de trifluorure de bore en solution dans l'éther éthylique et laisser sous agitation pendant 3 heures. Evaporer à sec après lavage à l'eau, et purifier le résidu obtenu sous forme de sirop sur colonne de silice. On obtient ainsi, 1,64 g d'un mélange d'anomères α et β et d'éthyl-2,4,6-tri-O-benzoyl-3-désoxy-1-thio-D-*ribo*-hexopyranoside.

Ce composé est utilisé sans procéder à une autre purification.
Rendement : 70 %

**PREPARATION II**

**Ethyl 2,4,6-tri-O-benzyl-3-désoxy-1-thio-D-*ribo*-hexopyranoside (Composé de formule V)**

Dissoudre 5,82 mmole du composé obtenu à la Préparation I, dans un mélange de méthanol et de dichlorométhane (1:1 V/V). Ajouter 0,90 mmole de méthanolate de sodium. Laisser le mélange réactionnel sous agitation pendant 3 heures à 20°C puis neutraliser à l'aide d'une résine Dowex acide (AG 50 WX2). Filtrer et évaporer à sec. Dissoudre le résidu dans 18 ml de diméthylformamide anhydre, puis ajouter à 0°C, 19,7 mmole d'hydrure de sodium et 17,0 mmole de bromure de benzoyle. Laisser sous agitation pendant 2 heures puis, ajouter 34,1 mmole de méthanol.

Evaporer les solvants réactionnels et purifier sur colonne de silice, pour obtenir 2,12 g du produit attendu sous forme de mélange d'anomères.
Rendement : 76 %

**PREPARATION III**

**1,6:2,3 di-anhydro-4-O-(2,4,6-tri-O-benzoyl-3-désoxy-β-D-*ribo*-hexopyranosyl)-β-D-mannopyranose (Composé de formule IX)**

Selon la méthode décrite par G.H. Veeneman et J.H. van Boom dans Tetrahedron Letters (1990), 31, pp. 275-278, dissoudre à -20°C, 8,53 mmole du produit de la Préparation I et 7,25 mmole de 1,6:2,3-di-anhydro-β-D-mannopyranose dans 220 ml de toluène en présence d'un tamis moléculaire et de 21,3 mmole de N-iodosuccinimide, puis ajouter goutte à goutte 1,7 mmole d'une solution d'acide trifluorométhane sulfonique 0,04 M. Laisser le milieu réactionnel sous agitation pendant 2,5 heures, filtrer et purifier sur colonne de silice pour obtenir 3,07 g de 1,6:2,3-di-anhydro-4-O-(2,4,6-tri-O-benzoyl-3-désoxy-β-D-*ribo*-hexopyranosyl)-β-D-mannopyranose.

Cristalliser dans un mélange d'acétate d'éthyle et d'hexane (90:10 V/V).
Rendement : 65 %
Point de fusion : 153°C
$[\alpha]_D^{20}$ = -12° (C = 1,10 dans $CH_2Cl_2$)

**PREPARATION IV**

**1,6:2,3 di-anhydro-4-O-(3-désoxy-β-D-*ribo*-hexopyranosyl)-β-D-mannopyranose (Composé de formule X)**

Soumettre le 1,6:2,3-di-anhydro-4-O-(2,4,6-tri-O-benzoyl-3-désoxy-β -D-*ribo*-hexopyranosyl)-β-D-mannopyranose à une débenzoylation à l'aide de méthanolate de sodium, pour obtenir le 1,6:2,3 di-anhydro-4-O-(3-désoxy-β-D-*ribo*-hexopyranosyl)-β-D-mannopyranose.
Rendement : 95 %
$[\alpha]_D^{20}$ = -46° (C = 1,02 dans $CH_3OH$)

**PREPARATION V**

**3-O-acétyl-1,6-anhydro-2-O-benzyl-4-O-(benzyl-2-O-acétyl-3-désoxy-β-D-*ribo*-hexopyranosyluronate)-β-D-glucopyranose (Composé de formule VI)**

Ce composé a été préparé à partir du 1,6:2,3-di-anhydro-4-O-(3-désoxy-β-D-*ribo*-hexopyranosyl)-β-D-mannopyranose, composé décrit dans la préparation IV, en utilisant des procédés analogues à ceux déjà décrits par M. Petitou et C.A.A. van Boeckel dans "Chemical Synthesis of heparin fragments and analogues" pp. 203-210 - Progress in the Chemistry of Organic Natural Products, Ed. Springer Verlag Wien - N.Y. (1992), et notamment : formation du 1,6:2,3-di-anhydro-4-O-(3-désoxy-4,6-O-isopropylidène-β-D-*ribo*-hexopyranosyl)-β-D-mannopyranose, réaction avec le benzylate de sodium, acétylation, élimination du radical isopropylidène à l'aide de l'acide acétique, silylation, réaction avec l'anhydride lévulinique, oxydation selon les conditions de Jones, estérification à l'aide de bromure de benzyle, et ensuite traitement par l'hydrazine.

Les différents stades sont indiqués ci-après.

STADE A

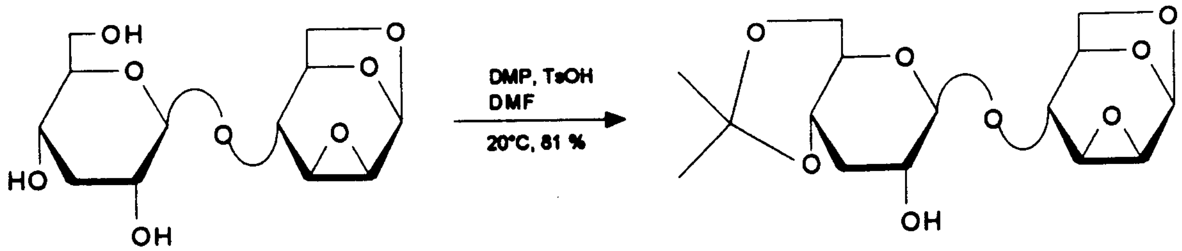


Ajouter dans une solution de 1,6:2,3-di-anhydro4-O-(3-désoxy-β-D-*ribo*-hexopyranosyl)-β-D-mannopyranose dans le diméthylformamide (5 mmole dans 35 ml) goutte à goutte et sous argon, 1,5 mmole d'acide p-toluène sulfonique (l'acide camphosulfonique peut aussi être utilisé) et 250 mmole de 2,2-diméthoxypropane. Laisser sous agitation pendant 2,5 heures puis ajouter 1,8 mmole de triéthylamine. Diluer avec du dichlorométhane, laver à l'eau, sécher sur sulfate de sodium anhydre, filtrer et évaporer à sec pour obtenir le 1,6:2,3-di-anhydro-4-O-(3-désoxy-4,6-O-isopropylidene-β-D-*ribo*-hexopyranosyl)-β-D-mannopyranose. Utiliser ce composé à l'étape suivante sans procéder à une purification.
Rendement : 81 %

STADE B

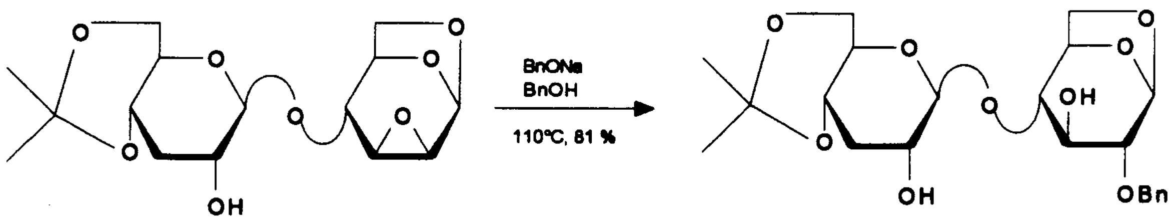


Ajouter à 5,0 mmole du composé obtenu au Stade A, 25 mmole de benzylate de sodium (solution 1 M dans l'alcool benzylique). Chauffer à 110°C pendant 30 mn. Refroidir, neutraliser à l'aide d'une résine Dowex acide (AG 50 WX2), filtrer et éliminer l'alcool benzylique en évaporant sous vide. Purifier le résidu sur colonne de silice en utilisant comme éluant un mélange de toluène et d'acétone (3:1 V/V). Le produit attendu est obtenu sous forme de sirop.
Rendement : 81 %

STADE C

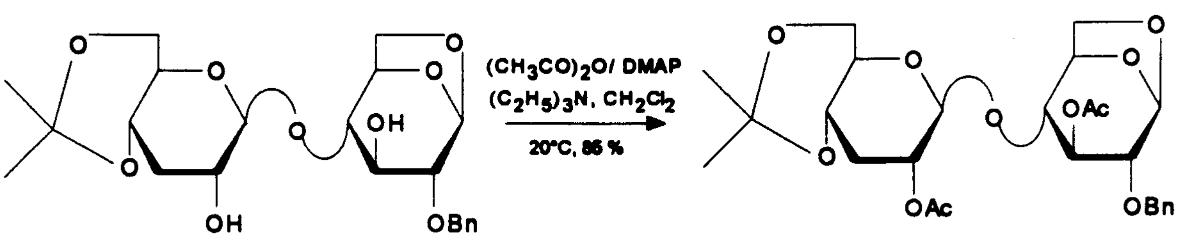


Dissoudre 4,90 mmole du composé obtenu au Stade B dans 22 ml de dichlorométhane, refroidir à 0°C et ajouter 19,6 mmole d'anhydride acétique, 1,96 mmole de 4-diméthylaminopyridine et 9,81 mmole de triéthylamine. Agiter à température ambiante pendant 45 mn. Ajouter du méthanol et maintenir sous agitation pendant encore 30 mn. Diluer ensuite le milieu réactionnel avec du dichlorométhane, laver avec une solution aqueuse de $KHSO_4$, puis à l'eau, sécher sur sulfate de sodium anhydre, évaporer à sec pour obtenir le produit attendu sous forme de sirop. Purifier ensuite sur colonne de silice en utilisant comme éluant un mélange de toluène et d'acétone (9:1 V/V)
Rendement : 85 %

STADE D

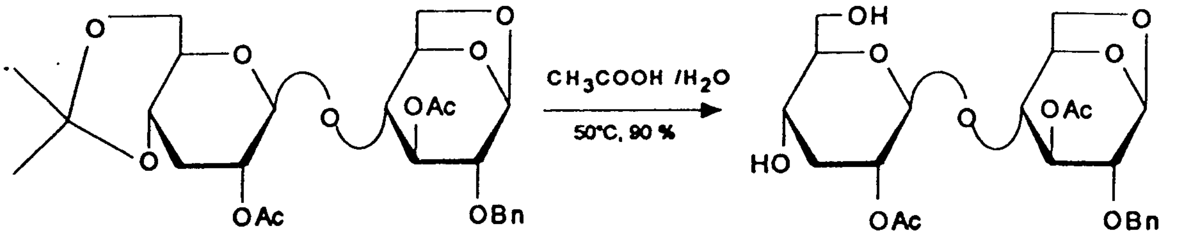


Dissoudre 4,11 mmole du composé obtenu au stade précédent, dans 2,2 ml de 1,1-dichloroéthane et ajouter 123 ml d'une solution aqueuse d'acide acétique (70 %). Laisser sous agitation pendant 35 mn à 50°C. Concentrer, ajouter du toluène et évaporer pour obtenir le produit attendu sous forme de sirop. Purifier sur colonne de silice en utilisant comme éluant un mélange de cyclohexane et d'acétone (1:1 V/V).
Rendement : 90 %

STADE E

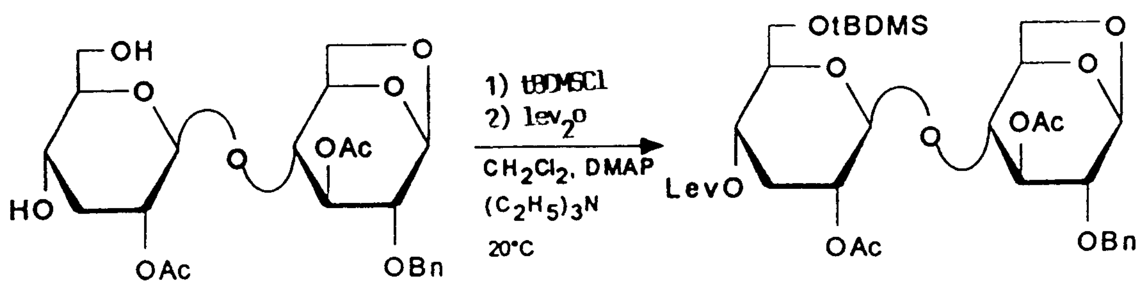


Dissoudre 3,61 mmole du composé obtenu au stade D dans 3,3 ml de dichlorométhane et ajouter 1,42 mmole de 4-diméthylaminopyridine, 10,82 mmole de triéthylamine et 5,41 mmole de chlorure de tertiobutyl diméthyl silyle.

Laisser sous agitation pendant environ 1 heure à 20°C puis ajouter 43 ml de dichlorométhane anhydre et 10,8 mmole d'anhydride lévulinique. Laisser sous agitation pendant 2 heures, puis ajouter 150 ml de dichlorométhane, laver d'abord avec une solution aqueuse de $KHSO_4$, puis avec une solution aqueuse de $NaHSO_4$, sécher sur sulfate de sodium anhydre, filtrer et évaporer jusqu'à l'obtention d'un sirop brun.

Utiliser le produit tel quel au stade suivant.

STADE F

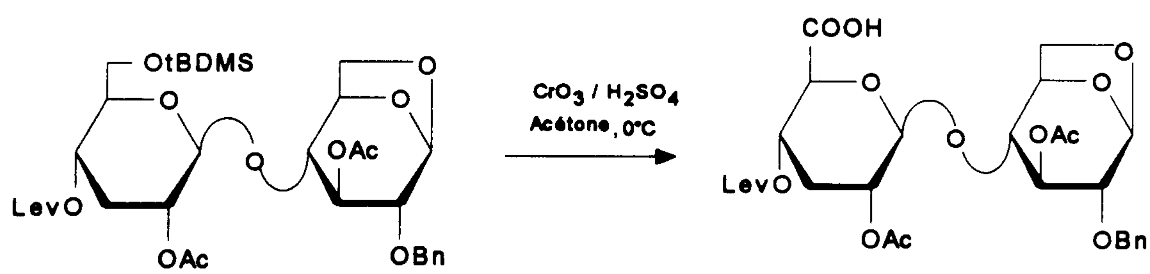


Dissoudre 2,51 g du sirop obtenu au stade précédent dans 26 ml d'acétone, refroidir à 0°C puis ajouter 9,56 mmole de trioxyde de chrome et 4,2 ml d'une solution d'acide sulfurique 3,5 M.

Laisser sous agitation à température ambiante pendant 4 heures. Ajouter ensuite 250 ml de dichlorométhane, laver à l'eau, sécher sur sulfate de sodium anhydre, filtrer et évaporer jusqu'à l'obtention d'un sirop brun.

STADE G

Préparation du 3-O-acétyl-1,6-anhydro-2-O-benzyl-4-O-(benzyl-2-O-acétyl-3-désoxy-4-O-lévulinyl-β-D-*ribo*-hexopyranosyluronate)-β-D-glucopyranose (Composé de formule XI)

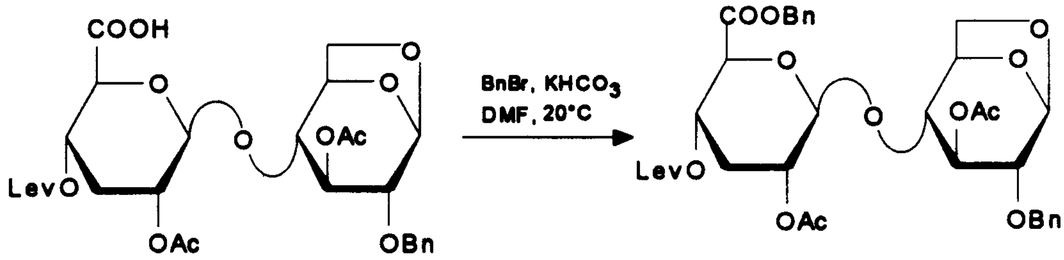


Dissoudre 2,15 g de produit obtenu au stade précédent dans 22 ml de diméthylformamide anhydre et ajouter sous argon 7,22 mmole de bicarbonate de potassium et 10,83 mmole de bromure de benzyle. Laisser sous agitation pendant 3 heures, puis ajouter 0,5 ml de méthanol et continuer l'agitation pendant 1 heure à température ambiante.

Diluer le milieu réactionnel avec de l'acétate d'éthyle, laver à l'eau, sécher sur sulfate de sodium anhydre, filtrer et évaporer jusqu'à l'obtention d'un sirop brun.

Rendement global des stades E, F, G : 81 %

STADE H

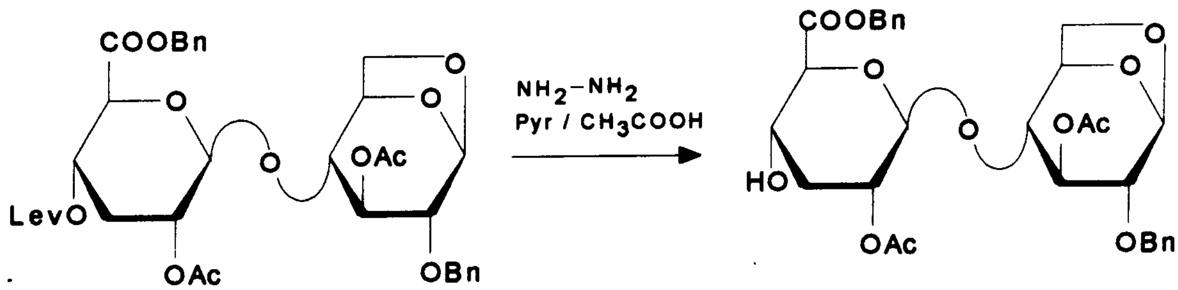


Dissoudre dans 13 ml de pyridine 3,61 mmole de 3,O-acétyl-1,6-anhydro-2-O-benzyl-4-O-(benzyl-2-O-acétyl-3-désoxy-4-O-levulinyl-β-D-*ribo*-hexopyranosyluronate)-β-D-glucopyranose, composé obtenu au stade G. Refroidir à 0°C et ajouter 18,1 mmole d'hydrazine [solution 1 M dans un mélange de pyridine et d'acide acétique (3:2 V/V)]. Laisser le mélange réactionnel sous agitation pendant 15 mn à température ambiante. Concentrer, ajouter du dichlorométhane, laver avec une solution aqueuse de $KHSO_4$, puis à l'eau et ensuite avec une solution aqueuse de $NaHCO_3$ et à nouveau à l'eau. Sécher sur sulfate de sodium, filtrer et évaporer à sec pour obtenir un sirop brun. Le 3-O-acétyl-1,6-anhydro-2-O-benzyl-4-O-(benzyl-2-O-acétyl-3-désoxy-β-D-*ribo*-hexopyranosyluronate)-β-D-glucopyranose est purifié sur colonne de silice en utilisant comme solvant un mélange de cyclohexane et d'acétone (2:1 V/V).

Rendement : 86 %

$[\alpha]_D^{20}$ = -78° (C= 0,7 dans $CH_2Cl_2$)

**PREPARATION VI**

**O-(benzyl-2-O-acétyl-3-désoxy-4-O-lévulinyl-β-D-*ribo*-hexopyranosyluronate)-(1→4)-3,6-di-O-acétyl-2-O-benzyl-D-glucopyranosyl) trichloroacétimidate (Composé de formule XIIa)**

Ce composé a été préparé à partir du 3-O-acétyl-1,6-anhydro-2-O-benzyl-4-O-(benzyl-2-O-acétyl-3-désoxy-4-O-lévulinyl-β-D-*ribo*-hexopyranosyl-uronate)-β-D-glucopyranose, composé décrit dans la Préparation V, selon des méthodes connues et notamment acétolyse, déprotection anomérique et formation de l'imidate. Les différents stades sont donnés ci-après.

STADE A

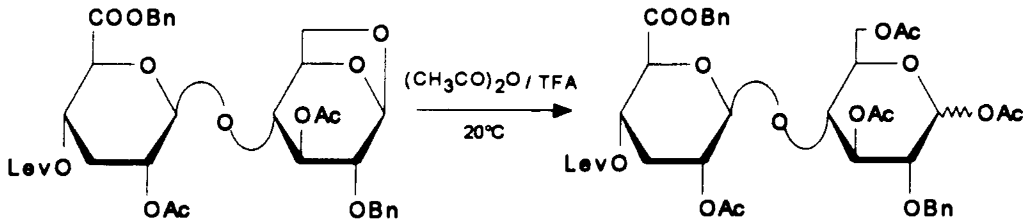


A 1,02 mmole de produit obtenu à la Préparation V, ajouter 102 mmole d'anhydride acétique et 10,2 mmole de trifluoroacétamide. Laisser le mélange pendant 2 heures sous agitation et sous argon. Evaporer ensuite jusqu'à l'obtention d'un sirop brun et purifier sur colonne de silice en utilisant comme éluant un mélange de cyclohexane et d'acétate d'éthyle (2:3 V/V).

STADE B

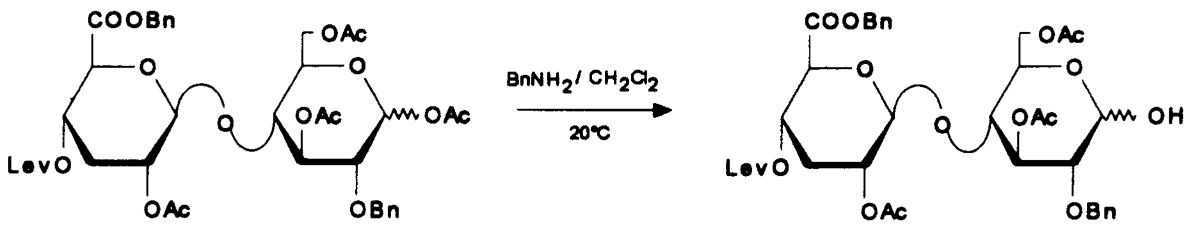


Dissoudre 1,52 mmole du composé obtenu au stade précédent dans le dichlorométhane et ajouter 57,9 mmole de benzylamine. Laisser sous agitation pendant 4 heures à température ambiante puis abandonner à -20°C pendant une nuit. Ajouter de l'éther éthylique et laver avec une solution aqueuse d'acide chlorhydrique 1N. Extraire avec du dichlorométhane, sécher sur sulfate de sodium anhydre et évaporer jusqu'à l'obtention d'un sirop brun. Purifier sur colonne de silice en utilisant comme éluant un mélange de toluène et d'acétone (5:1 V/V).

STADE C

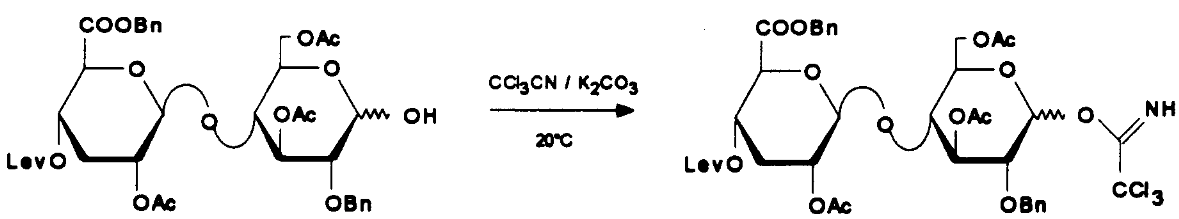


Utiliser 0,246 mmole du disaccharide obtenu au stade précédent en solution dans le dichlorométhane. Ajouter sous argon 0,39 mmole de carbonate de potassium et 1,23 mmole de trichloroacétonitrile. Laisser sous agitation pendant 16 heures, filtrer et évaporer à sec. Le O-(benzyl-2-O-acétyl-3-désoxy-4-O-lévulinyl-β-D-*ribo*-hexopyranosyluronate)-(1 → 4)-3,6-di-O-acétyl-2-O-benzyl-D-glucopyranosyl) trichloroacétimidate est obtenu sous forme d'un mélange d'anomères.

Rendement : 62 % (global)

**PREPARATION VII**

**1,6:2,3-di-anhydro-4-O-(3-désoxy-α-D-*ribo*-hexopyranosyl)-β-D-mannopyranose (Composé de formule XV - mélange gluco et ido)**

STADE A

1,6:2,3-di-anhydro-4-O-(3-désoxy-6-ido-β-D-*ribo*-hexopyranosyl)-β-D-mannopyranose

A une solution de 1,6:2,3-di-anhydro-4-O-(3-désoxy-β-D-*ribo*-hexopyranosyl)-β-D-mannopyranose (22,81 mmole) dans 400 ml d'un mélange de dichlorométhane et d'acétonitrile, ajouter 68,43 mmole de triphénylphosphine, 68,43 mmole d'imidazole et 29,65 mmole d'iode. Laisser sous agitation à 70°C pendant 4 heures. Evaporer la solution et purifier le sirop ainsi obtenu sur colonne de silice, en utilisant comme solvant un mélange de dichlorométhane et d'acétone (10:1 V/V). On obtient ainsi le 1,6:2,3-di-anhydro-4-O-(3-désoxy-6-ido-β-D-*ribo*-hexopyranosyl)-β-D-mannopyranose.
Rendement : 72 %

STADE B

Dissoudre 23,4 mmole de 1,6:2,3-di-anhydro-4-O-(3-désoxy-6-ido-β-D-*ribo*-hexopyranosyl)-β-D-mannopyranose dans 150 ml de méthanol et ajouter 42 mmole de méthylate de sodium en solution dans du méthanol (1M). Chauffer le mélange pendant 9 heures à 80°C, refroidir et purifier sur colonne Sephadex LH-20, en éluant avec un mélange de dichlorométhane et de méthanol (1:1 V/V). On obtient ainsi le 1,6:2,3-di-anhydro-4-O-(3-désoxy-5,6-exomethylène-β-D-*ribo*-hexopyranosyl)-β-D-mannopyranose. Dissoudre 13,3 mmole de ce composé dans 100 ml de tétrahydrofurane et ajouter goutte à goutte à 20°C, 54,4 mmole de diborane en solution dans le tétrahydrofurane. Laisser pendant deux heures sous agitation puis ajouter 65,28 mmole d'éthanol. Laisser sous agitation pendant une heure, puis ajouter 24 ml d'une solution d'hydroxyde de sodium 3 M et 24 ml d'une solution de peroxyde d'hydrogène à 30 %. Chauffer à 50°C, neutraliser sur résine Dowex acide (AG 50WX4) et évaporer à sec pour obtenir du 1,6:2,3-di-anhydro-4-O-(3-désoxy-α-D-*ribo*-hexopyranosyl)-β-D-mannopyranose sous forme d'un mélange de gluco et d'ido disaccharides (1:6,25).

**PREPARATION VIII**

**3-O-acétyl-1,6-anhydro-2-O-benzyl-4-O-(benzyl-2-O-acétyl-3-désoxy-4-O-lévulinyl-α-L-*lyxo*-hexopyranosyluronate)-β-D-glucopyranose (Composé de formule XVI)**

Le 3-O-acétyl-1,6-anhydro-2-O-benzyl-4-O-(benzyl-2-O-acétyl-3-désoxy -4-O-lévulinyl-α-L-*lyxo*-hexopyranosyluronate)-β-D-glucopyranose est préparé à partir du composé décrit dans la Préparation VII, en mettant en oeuvre des procédés classiques [G. Jaurand et *al* (Bioorganic and Medicinal Chemistry Letters (1992), 2 (n°9), pp. 897-900) ; J. Basten et *al*, (Bioorganic and Medicinal Chemistry Letters (1992), 2 (n°9), pp. 901-904); J. Basten et *al*, (Bioorganic and Medicinal Chemistry Letters (1992), 2 (n°9), pp. 905-910)] notamment, formation de deux 4'-6'-isopropylidènes correspondants, purification et séparation sur colonne de silice des isomères gluco et ido, réaction avec le benzylate de sodium, acétylation, élimination du radical isopropylidène, silylation sélective, réaction avec l'anhydride lévulinique, oxydation selon les conditions de Jones, et estérification à l'aide de bromure de benzyle.
Rendement : 45 %

Les différents stades sont donnés ci-après.

STADE A

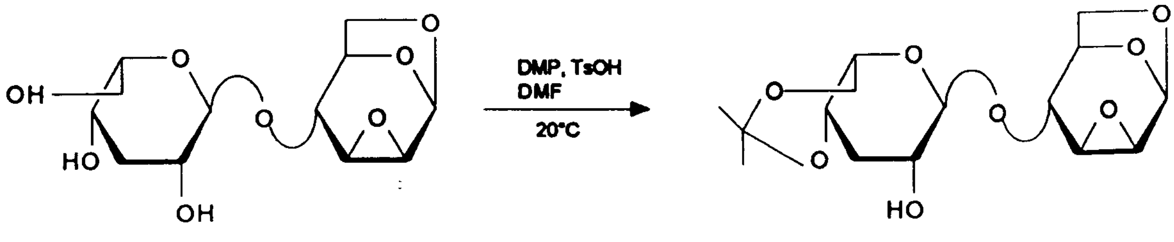


Dissoudre 3,6 mmole de 1,6:2,3-di-anhydro-4-O-(3-désoxy-α-D-*ribo*-hexopyranosyl)-β-D-mannopyranose dans 25 ml de diméthylformamide et ajouter 21,64 mmole de diméthoxypropane et 3,96 mmole d'acide p-toluènesulfonique

(l'acide camphorsulfonique peut aussi être utilisé). Laisser sous agitation pendant 1 heure 30 mn et introduire 5,94 mmole de triéthylamine. Concentrer jusqu'à l'obtention d'un sirop et purifier sur colonne de silice en utilisant comme éluant un mélange de toluène et d'acétate d'éthyle (2:3 V/V).
Rendement : 70 %

STADE B

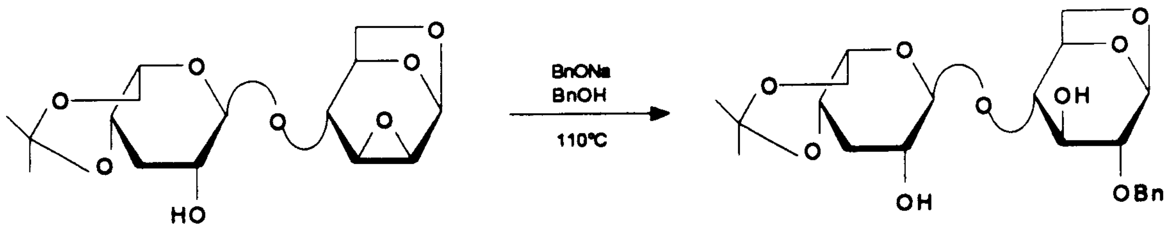


A 6,81 mmole du composé obtenu au stade A, ajouter 29,7 mmole de benzylate de sodium en solution dans l'alcool benzylique (1 M). Chauffer à 110°C sous agitation pendant 1 heure. Diluer ensuite avec 200 ml de dichlorométhane, puis neutraliser à l'aide d'une résine Dowex acide (AG 50 WX4), filtrer et évaporer sous vide pour obtenir un sirop brun. Purifier sur colonne de silice en utilisant comme éluant un mélange de dichlorométhane et d'acétone (10:1 V/V).
Rendement : 85 %

STADE C

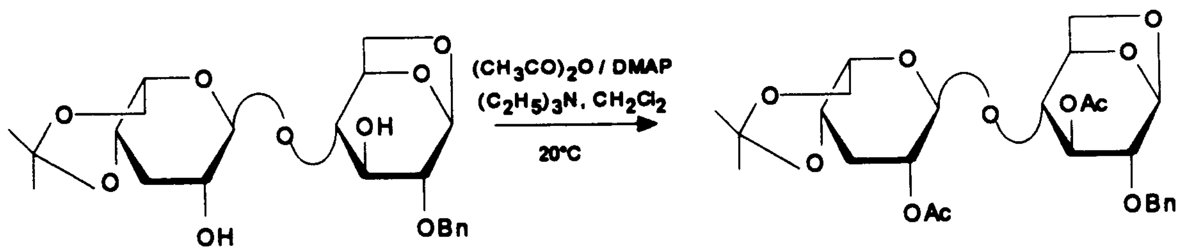


Dissoudre 5,76 mmole du composé obtenu au stade B dans du dichlorométhane et ajouter 3,92 mmole de 4-diméthylaminopyridine, 72,27 mmole de triéthylamine et 65,7 mmole d'anhydride acétique. Laisser sous agitation à température ambiante pendant 2 heures 30 mn puis ajouter 100 ml de dichlorométhane, laver avec une solution aqueuse de $KHSO_4$ à 10 %, sécher sur sulfate de sodium anhydre, puis filtrer.

Concentrer et purifier le sirop ainsi obtenu sur colonne de silice en utilisant comme éluant un mélange de dichlorométhane et d'acétone (10:1 V/V).
$[\alpha]_D^{20}$ = -102° (C= 1,37 dans $CH_2Cl_2$)

STADE D

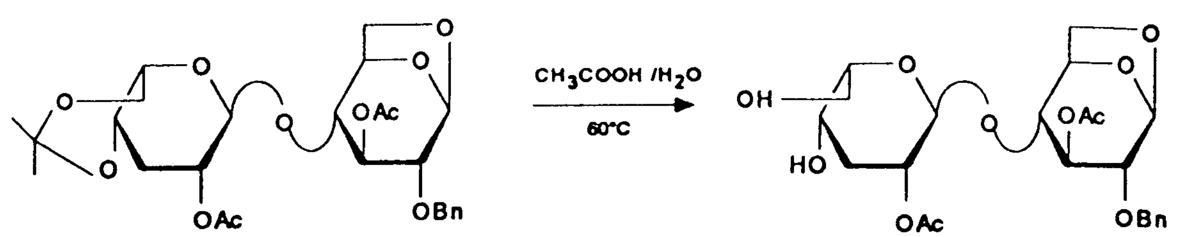


A 5,74 mmole du composé obtenu au stade C, ajouter 25 ml d'une solution aqueuse d'acide acétique à 70 %. Chauffer à 80°C pendant 6 heures. Concentrer en évaporant en présence de toluène pour obtenir une poudre jaune.

STADE E

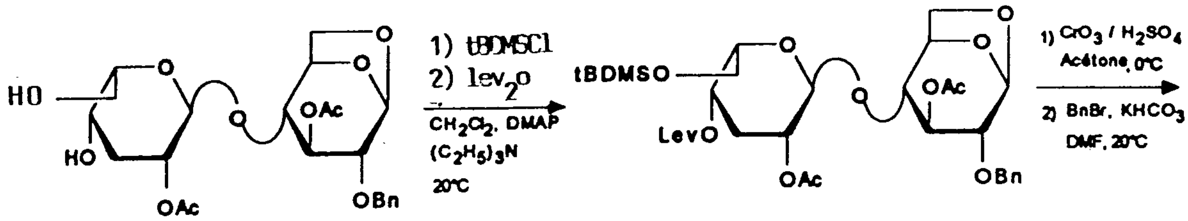


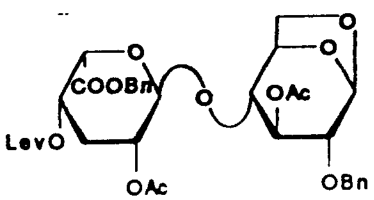

DMAP : 4-Diméthyl aminopyridine
tBDMSCl : Chlorure de tertiobutyl diméthyl silyle
BnBr : Bromure de benzyle
$lev_2O$ : Anhydride lévulinique
DMF : Diméthylformamide
Bn : radical benzyle
Ac : radical acétyle
Lev : radical lévulinyle
tBDMSI : radical tertiobutyl diméthyl silyle

En utilisant le produit obtenu au stade précédent, procéder comme il est décrit dans la préparation V, stades E, F et G pour obtenir le 3-O-acétyl-1,6-anhydro-2-O-benzyl-4-O-(benzyl-2-O-acétyl-3-désoxy-4-O-lévulinyl-α-L-*lyxo*-hexopyranosyluronate)-β-D-glucopyranose.
Rendement global : 59 %

**PREPARATION IX**

**Méthyl-3,6-di-O-acétyl-2-O-benzyl-4-O-(benzyl-2-O-acétyl-3-désoxy-α-L-*lyxo*-hexopyranosyluronate)-β-D-glucopyranoside (Composé de formule III)**

Soumettre le disaccharide obtenu à la préparation VIII à une acétolyse, traiter avec de la benzylamine, faire réagir avec le réactif de Vilsmeïer, puis, avec du méthanol en présence de carbonate d'argent. Ensuite, traiter le composé obtenu avec de l'hydrazine pour obtenir le méthyl-3,6-di-O-acétyl-2-O-benzyl-4-O-(benzyl-2-O-acétyl-3-désoxy-α-L-*lyxo*-hexopyranosyluronate)-β-D-glucopyrano side. Les différents stades sont donnés ci-après :

STADE A

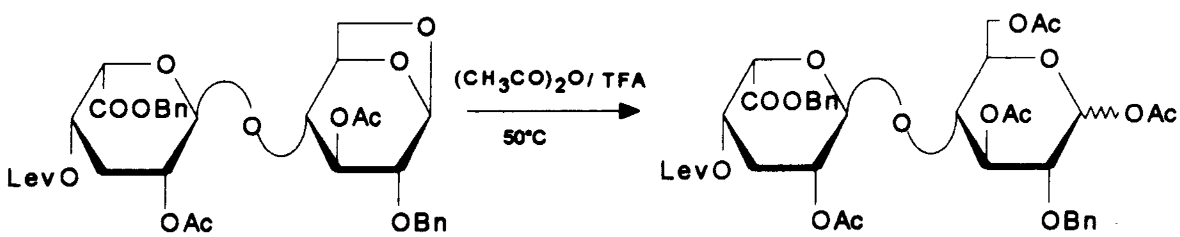


Ajouter à 1,02 mmole du disaccharide obtenu à la préparation VIII, 102 mmole d'anhydride acétique et 10,2 mmole d'acide trifluoroacétique, chauffer à 50°C pendant 2 heures sous argon. Evaporer ensuite jusqu'à l'obtention d'un sirop brun et purifier comme il est décrit dans la préparation VI, Stade A, pour obtenir le produit attendu.
Rendement : 92 %

STADE B

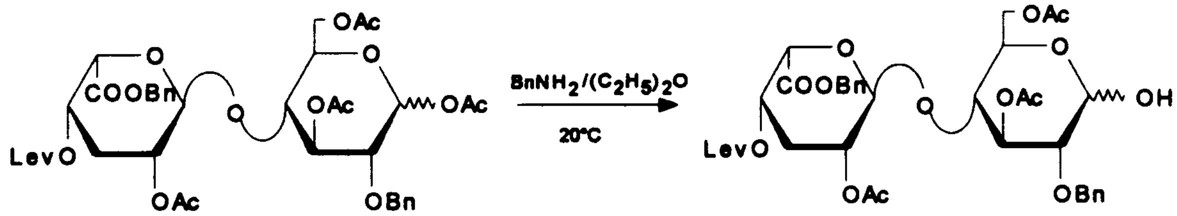


Dissoudre 1,52 mmole du composé obtenu au Stade A dans l'éther éthylique anhydre et ajouter 57,9 mmole de benzylamine. Procéder comme il est décrit dans le Stade B de la préparation VI pour obtenir le produit attendu.
Rendement : 79 %

STADE C

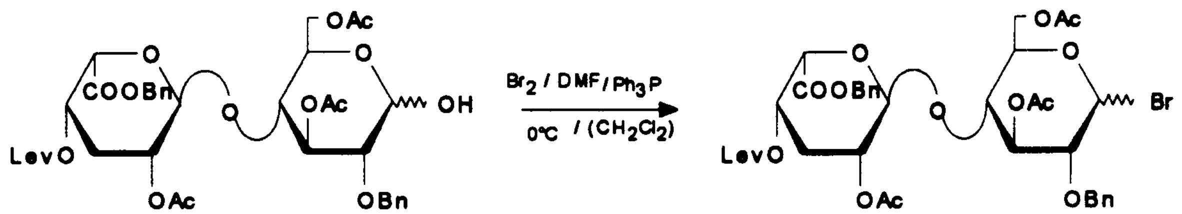


Préparer le réactif de Vilsmeïer en mélangeant à 0°C, 2,15 mmole de brome et 2,15 mmole de triphényl phosphine dans 5 ml de diméthylformamide. Filtrer le précipité blanc sous argon et ajouter en maintenant à la même température, 0,269 mmole du produit obtenu au Stade B, en solution dans 15 ml de dichlorométhane anhydre. Laisser le mélange réactionnel sous agitation pendant 2 jours à température ambiante. Diluer ensuite avec du dichlorométhane, laver à l'eau refroidie à 0°C jusqu'à l'obtention d'un pH 6, sécher sur sulfate de sodium anhydre et concentrer jusqu'à l'obtention d'un sirop. Purifier sur colonne de silice en utilisant un mélange de dichlorométhane et d'éther éthylique (5:1 V/V).
Rendement : 77 %

STADE D

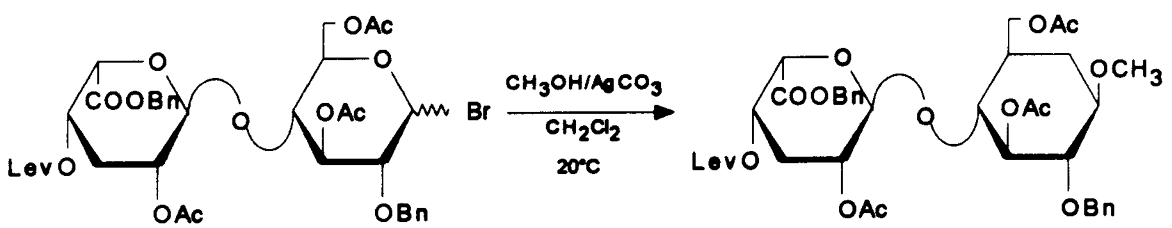


Mélanger 0,46 ml de méthanol, 0,34 mmole de carbonate d'argent et 150 mg de sulfate de calcium dans 3 ml de dichlorométhane pendant une heure sous atmosphère d'argon à 0°C. Dissoudre 0,227 mmole du composé obtenu au Stade C dans 8 ml de dichlorométhane. Ajouter cette solution goutte à goutte au mélange réactionnel et laisser sous agitation à 20°C pendant 20 heures. Protéger de la lumière. Diluer ensuite avec du dichlorométhane, filtrer et concentrer pour obtenir le méthyl-3,6-di-O-acétyl-2-O-benzyl-4-O-(benzyl-2-O-acétyl-3-désoxy-4-O-lévulinyl-α-L-*lyxo*-hexopyranosyluronate)-β-D-glucopyranoside sous forme de sirop. Purifier sur colonne de silice en utilisant comme éluant un mélange de dichlorométhane et d'éther éthylique (5:1 V/V).
Rendement : 75 %
$[\alpha]_D^{20}$ = +27° (C= 1,18 dans $CH_2Cl_2$)

STADE E

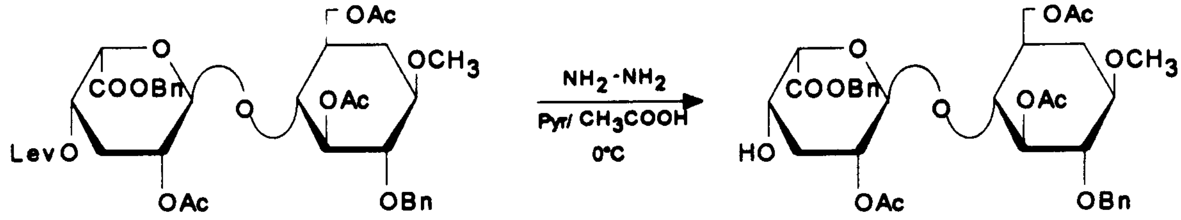


Dissoudre 0,276 mmole du produit obtenu au stade précédent dans 2 ml de pyridine refroidie à 0°C et ajouter 1,38 ml d'une solution 1 M d'hydrate d'hydrazine dans un mélange de pyridine et d'acide acétique (3:2 V/V). Concentrer ensuite le mélange réactionnel, ajouter du chlorure de méthylène, laver avec une solution aqueuse de $KHSO_4$, sécher sur sulfate de sodium anhydre, filtrer et concentrer jusqu'à l'obtention d'un sirop. Purifier sur colonne de silice en utilisant un mélange de chlorure de méthylène et d'éther éthylique (3:1 V/V)
Rendement : 87 %
$[\alpha]_D^{20}$ = +10° (C= 0,99 dans $CH_2Cl_2$)

**PREPARATION X**

**O-(2,4,6-tri-O-benzyl -3-désoxy-α-D-*ribo*-hexopyranosyl)-(1 → 4)-O-(benzyl-2-O-acétyl-3-désoxy-β-D-*ribo*-hexopyranosyluronate)-(1 → 4)-3-O-acétyl-1,6-anhydro-2-O-benzyl-β-D-glucopyranose (Composé de formule VII)**

Dissoudre 2,03 mmole d'éthyl-2,4,6-tri-O-benzyl-3-désoxy-1-thio-D-*ribo*-hexopyranoside (Préparation II) et 1,69 mmole de 3-O-acétyl-1,6-anhydro-2-O-benzy-4-O-(benzyl-2-O-acétyl-3-désoxy-β-D-*ribo*-hexopyranosyluronate)-β-D-glucopyranose (Préparation V) dans du dichlorométhane, ajouter à 20 °C du tamis moléculaire, puis, 5,07 mmole de trifluorométhane sulfonate d'argent et 1,52 mmole de brome. Laisser sous agitation pendant 45 minutes et ensuite, filtrer le mélange réactionnel, laver à l'eau, évaporer à sec.

Purifier sur colonne de silice pour obtenir le produit attendu.
Rendement : 35 %

**PREPARATION XI**

**O-(6-O-acétyl-2,4-di-O-benzyl-3-désoxy-α-D-*ribo*-hexopyranosyl)-(1 → 4)-O-(benzyl-2-O-acétyl-3-désoxy-β-D-*ribo*-hexopyranosyluronate)-(1 → 4)-3,6-di-O-acétyl-2-O-benzyl-β-D-glucopyranosyl trichloroacétimidate (Composé de formule II)**

Soumettre à une acétolyse, 0,51 mmole du composé obtenu à la Préparation X, en solution dans un mélange d'acide trifluoroacétique et d'anhydride acétique, traiter ensuite avec de la benzylamine dans l'éther éthylique puis, avec du trichloroacétonitrile dans le dichlorométhane en présence de carbonate de potassium, pour obtenir le produit attendu.
Rendement : 50 %

EXEMPLE 1

**Méthyl O-(3-désoxy-2,4,6-tri-O-sulfo-α-D-*ribo*-hexopyranosyl)-(1 → 4)-O-(3-désoxy-2-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-α-L-*lyxo*-hexopyranosyluronate)-(1 → 4)-2,3,6-tri-O-sulfo-β-D-glucopyranoside tridecakis sel de sodium**

STADE A

Méthyl O-(6-O-acétyl-2,4-di-O-benzyl-3-désoxy-α-D-*ribo*-hexopyranosyl)-(1 → 4)-O-(benzyl-2-O-acétyl-3-désoxy-β-D-*ribo*-hexopyranosyluronate)-(1 → 4)-O-(3,6-di-O-acétyl-2-O-benzyl-α-D-glucopyranosyl)-(1 → 4)-O-(benzyl-2-O-acétyl-3-désoxy-α-L-*lyxo*-hexopyranosyluronate)-(1 → 4)-O-3,6-di-O-acétyl-2-benzyl-β-D-glucopyranoside

Dissoudre 0,121 mmole du composé décrit dans la Préparation XI et 0,093 mmole du composé de la Préparation IX dans 3,2 ml de dichlorométhane. Refroidir à - 20°C en présence de tamis moléculaire et sous atmosphère d'argon,

ajouter 0,470 ml d'une solution de trifluorométhane sulfonate de triméthylsilyle en solution dans le dichlorométhane. Laisser sous agitation à -20°C pendant 1 heure, puis, filtrer le milieu réactionnel, laver à l'eau, évaporer et purifier sur colonne Sephadex LH-20 en utilisant comme solvant un mélange de dichlorométhane et de méthanol (1:1 V/V), puis sur une colonne de silice en utilisant comme solvant un mélange de cyclohexane et d'acétate d'éthyle (3:2 V/V), pour obtenir le produit attendu.
Rendement : 62 %

STADE B

Dissoudre 0,052 mmole de produit obtenu au stade précédent, dans un mélange de dichlorométhane (0,43 ml) et de méthanol (1,7 ml). Ajouter ensuite 85 mg de palladium à 10 % sur charbon et laisser le mélange pendant 4 heures à 20°C et sous légère pression d'hydrogène. Filtrer et évaporer à sec pour obtenir le méthyl O-(6-O-acétyl-3-désoxy-α-D-*ribo*-hexopyranosyl)-(1 → 4)-O-(2-O-acétyl-3-désoxy-β-D-*ribo*-hexopyranosyl uronique acide)-(1 → 4)-O-(3,6-di-O-acétyl-α-D-glucopyranosyl)-(1 → 4)-O-(2-O-acétyl-3-désoxy-α-L-*lyxo*-hexopyranosyl uronique acide)-(1 → 4)-3,6-di-O-acétyl-β-D-glucopyranoside. Dissoudre ce composé dans 0,96 ml d'éthanol, refroidir à 0°C puis ajouter 0,30 ml d'hydroxyde de sodium 5 M. Laisser sous agitation à 0°C pendant 5 heures, purifier sur colonne Sephadex G-25, en utilisant comme éluant de l'eau. Evaporer à sec pour obtenir le méthyl O-(3-dèsoxy-α-D-*ribo*-hexopyranosyl)-(1 → 4)-O-(3-désoxy-β-D-*ribo*-hexopyranosyl uronique acide)-(1 → 4)-O-(α-D-glucopyranosyl)-(1 → 4)-O-(3-désoxy-α-L-*lyxo*-hexopyranosyl uronique acide-(1 → 4)-β-D-glucopyranoside.

Dissoudre ce composé dans 5 ml de diméthylformamide, évaporer à sec, puis dissoudre à nouveau sous atmosphère d'argon dans 2,6 ml de diméthylformamide anhydre. Ajouter 302 mg de complexe trioxyde de soufre triéthylamine et agiter pendant 20 heures à 55°C. Refroidir le milieu réactionnel, ajouter 491 mg de bicarbonate de sodium en solution dans l'eau et laisser sous agitation pendant 3 heures.

Purifier sur colonne Sephadex G-25 en utilisant comme solvant de l'eau puis lyophiliser pour obtenir le méthyl O-(3-désoxy-2,4,6-tri-O-sulfo-α-D-*ribo*-hexopyranosyl-(1 → 4)-O-(3-désoxy-2-O-sulfo-β-D-*ribo*-hexopyranosyluronate)-(1 → 4)-O-(2,3,6-tri-O-sulfo-α-D-glucopyranosyl-(1 → 4)-O-(3-désoxy-2-O-sulfo-α-L-*lyxo*-hexopyranosyluronate-(1 → 4)-2,3,6-tri-O-sulfo-β-D-glucopyranoside tridecakis sel de sodium.
Rendement : 65%
$[\alpha]_D^{20}$ = + 25° (C = 0,52 dans $H_2O$) - Lot 1
$[\alpha]_D^{20}$ = + 33° (C = 0,64 dans $H_2O$) - Lot 2

Spectre de résonance magnétique nucléaire du proton : (500 MHz, solvant $D_2O$)

H1 : 4,78 ppm J1-2 : 7,3 Hz ; H'1 : 5,22 ppm, J1'-2' : 1,8 Hz ; H"1 : 5,26 ppm, J1"-2" : 3,5 Hz; H'"1 : 4,78 ppm, J1'"-2'" : 5,4 Hz ; H"" : 5,16 ppm, J1""-2"" : 3,3 Hz.

## EXEMPLE 2

**Méthyl O-(3-désoxy-2,4,6-tri-O-sulfo-α-D-*ribo*-hexopyranosyl)-(1 → 4)-O-(3-désoxy-2-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-méthyl-α-L-*lyxo*-hexopyranosyluronate)-(1 → 4)-2,3,6-tri-O-sulfo-β-D-glucopyranoside dodecakis sel de sodium**

Ce composé a été préparé à partir du O-(6-O-acétyl-2,4-di-O-benzyl-3-désoxy-α-D-*ribo*-hexopyranosyl)-(1 → 4)-O(benzyl-2-O-acétyl-3-désoxy-β-D-*ribo*-hexopyranosyluronate)-(1 → 4)-3,6-di-O-acétyl-2-O-benzyl-D-glucopyranosyl trichloroacétamidate et du méthyl 3,6-di-O-acétyl-2-O-benzyl-4-O-(benzyl-2-O-méthyl 3-désoxy-α-L-*lyxo*-hexopyranosyluronate)-β-D-glucopyranoside selon le procédé décrit dans l'Exemple 1. Le méthyl 3,6-di-O-acétyl-2-O-benzyl-4-O-(benzyl-2-O-méthyl 3-désoxy-α-L-*lyxo*-hexopyranosyluronate)-β-D-glucopyranoside a été préparé à partir du 1,6:2,3-di-anhydro-4-O-(3-désoxy-D-*ribo*-hexopyranosyl)-β-D-mannopyranose, selon un procédé similaire à ceux décrits dans les Préparations VII, VIII et IX.
$[\alpha]_D^{20}$ = + 25° (C = 0,48 dans $H_2O$)

Spectre de résonance magnétique nucléaire du proton : (500 MHz, solvant $D_2O$)

H1 : 4,82 ppm J1-2 : 4,2 Hz ; H'1 : 5,08 ppm, J1'-2' : 1,0 Hz; H"1 : 5,27 ppm, J1"-2" : 3,7 Hz ; H'"1 : 4,76 ppm, J1'"-2'" : 7,7 Hz ; H""1 : 5,15 ppm, J1""-2"" : 3,5 Hz.

**EXEMPLE 3**

**Méthyl O-(3-désoxy-2,4-di-O-méthyl-6-O-sulfo-α-D-*ribo*-hexopyranosyl)-(1 → 4)-O-(3-désoxy-2-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-α-L-*lyxo*hexopyranosyluronate)-(1 → 4)-2,3,6-tri-O-sulfo-β-D-glucopyranoside undecakis sel de sodium**

Ce composé a été préparé à partir du O-(6-O-benzyl-2,4-di-O-méthyl-3-désoxy-α-D-*ribo*-hexopyranosyl)-(1 → 4)-O-(benzyl-2-O-acétyl-3-désoxy-β-D-*ribo*-hexopyranosyluronate)-(1 → 4)-3,6-di-O-acétyl-2-O-benzyl-D-glucopyranosyl trichloroacétamidate et du méthyl 3,6-di-O-acétyl-2-O-benzyl-4-O-(benzyl-2-O-acétyl 3-désoxy-α-L-*lyxo*-hexopyranosyluronate)-β-D-glucopyranoside selon le procédé décrit dans l'Exemple 1. Le O-(6-O-benzyl-2,4-di-O-méthyl-3-désoxy-α-D-*ribo*-hexopyranosyl)-(1 → 4)-O-(benzyl-2-O-acétyl-3-désoxy-β-*ribo*-hexopyranosyluronate)-(1 → 4)-3,6-di-O-acétyl-2-O-benzyl-D-glucopyranosyl trichloroacétimidate a été préparé à partir de l'éthyl 6-O-benzyl-2,4-di-O-méthyl-3-désoxy-1-thio-β-D-*ribo*-hexopyranoside et du 3-O-acétyl-1,6-anhydro-2-O-benzyl-4-O-(benzyl 2-O-acétyl-3-désoxy-β-D-*ribo*-hexopyranosyluronate)-β-D-glucopyranose, selon le procédé décrit dans la préparation X.
$[\alpha]_D^{20}$ = + 24° (C = 0,6 dans $H_2O$)

Spectre de résonance magnétique nucléaire du proton : (500 MHz, solvant $D_2O$)

H1 : 4,77 ppm, J1-2 : 5,0 Hz : H'1 : 5,22 ppm, J1'-2' : 2,2 Hz ; H"1 : 5,24 ppm, J1"-2" : 3,7 Hz : H"'1 : 4,76 ppm, J1"'-2"' : 7,5 Hz ; H""1 : 5,10 ppm, J1""-2"" : 3,6 Hz.

**EXEMPLE 4**

**Méthyl O-(3-désoxy-4-O-méthyl-2,6-di-O-sulfo-α-D-*ribo*-hexopyranosyl)-(1 → 4)-O-(3-désoxy-2-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-α-L-*lyxo*-hexopyranosyluronate)-(1 → 4)-2,3,6-tri-O-sulfo-β-D-glucopyranoside dodecalkis sel de sodium**

Ce composé a été préparé selon le procédé décrit dans l'exemple 1, à partir du composé décrit dans la Préparation IX, et du O-(2,6-di-O-benzyl-4-O-méthyl-3-désoxy-α-D-*ribo*-hexopyranosyl)-(1 → 4)-O-(benzyl-2-O-acétyl-3-désoxy -β-D-*ribo*-hexopyranosyluronate)-(1 → 4)-3,6-di-O-acétyl-2-O-benzyl-D-glucopyranosyl trichloroacétimidate. Ce dernier dérivé trisaccharidique a été préparé à partir de l'éthyl-2,6-di-O-benzyl-4-O-méthyl-3-désoxy-1-thio-D-*ribo*-hexopyranoside, et du composé décrit dans la Préparation V, selon le procédé décrit dans les Préparations X et XI.
$[\alpha]_D^{20}$ = + 22° (C = 0,54 dans $H_2O$)

Spectre de résonance magnétique nucléaire du proton : (500 MHz, solvant $D_2O$)

H1 : 4,78 ppm, J1-2 : 5,1 Hz : H'1 : 5,22 ppm, J1'-2' : 2,6 Hz : H"1 : 5,27 ppm, J1"-2" : 3,9 Hz : H"'1 : 4,75 ppm, J1"'-2"' : 7,9 Hz ; H""1 : 5,11 ppm, J1""-2"" : 3,5 Hz.

**EXEMPLE 5**

**Méthyl O-(3-désoxy-2,4,6-tri-O-sulfo-α-D-*ribo*-hexopyranosyl)-(1 → 4)-O-(3-désoxy-2-O-méthyl-β-D-*ribo*-hexopyranosyluronate)-(1 → 4)-O-(2,3,6-tri-O-sulfo-α-D-glucopyranosyl)-(1 → 4)-O-(3-désoxy-2-O-méthyl-α-L-*lyxo*-hexopyranosyluronate)-(1 → 4)-2,3,6-tri-O-sulfo-β-D-glucopyranoside undecakis sel de sodium**

Ce composé a été préparé à partir du méthyl 3,6-di-O-acétyl-2-O-benzyl-4-O-(benzyl-2-O-méthyl-3-désoxy-α-L-*lyxo*-hexopyranosyluronate)-β-D-glucopyranoside et du O-(6-O-acétyl-2,4-di-O-benzyl-3-désoxy-α-D-*ribo*-hexopyranosyl)-(1 → 4)-O-(benzyl-2-O-méthyl-3-désoxy-β-D-*ribo*-hexopyranosyluronate)-(1 → 4)-3,6-di-O-acétyl-2-O-benzyl-D-glucopyranosyl trichloroacétimidate, selon le procédé décrit dans l'Exemple 1. Le trisaccharide utilisé comme produit de départ a été préparé à partir du 3-O-acétyl-1,6-anhydro-2-O-benzyl-4-O-(benzyl-2-O-méthyl-3-désoxy-β-D-*ribo*-hexopyranosyluronate-β-D-glucopyrano se et du composé décrit dans la Préparation II, selon le procédé décrit dans les Préparations X et XI.
$[\alpha]_D^{20}$ = + 20° (C = 0,39 dans $H_2O$)

Spectre de résonance magnétique nucléaire du proton : (500 MHz, solvant $D_2O$)

H1 : 4,83 ppm, J1-2 : 3,0 Hz ; H'1 : 5,11 ppm, J1'-2' : 2,0 Hz; H"1 : 5,31 ppm, J1"-2" : 3,3 Hz ; H"'1 : 4,68 ppm, J1"'-2"' : 7,7 Hz ; H""1 : 5,18 ppm, J1""-2"" : 3,2 Hz.

**EXEMPLE 6**

**Méthyl O-(3-désoxy-2,4,6-tri-O-sulfo-α-D-*ribo*-hexopyranosyl)-(1 → 4)-O-(3-désoxy-2-O-méthyl-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-α-L-*lyxo*-hexopyranosyluronate)-(1 → 4)-2,3,6-tri-O-sulfo-β-D-glucopyranoside dodecakis sel de sodium**

Ce composé a été préparé selon le procédé décrit dans l'Exemple 1, à partir du O-(6-O-acétyl-2,4-di-O-benzyl-3-désoxy-α-D-*ribo*-hexopyranosyl)-(1 → 4)-O-(benzyl-2-O-méthyl-3-désoxy-β-D-*ribo*-hexopyranosyluronate)-(1 → 4)-3,6-di-O-acétyl-2-O-benzyl-D-glucopyranosyl trichloroacétimidate, et du composé décrit dans la Préparation IX.
$[\alpha]_D^{20}$ = + 30° (C = 0,40 dans $H_2O$)

Spectre de résonance magnétique nucléaire du proton : (500 MHz, solvant $D_2O$)

H1 : 4,77 ppm, J1-2 : 5,0 Hz ; H'1 : 5,20 ppm, J1'-2' : 2,5 Hz ; H"1 : 5,27 ppm, J1"-2" : 4,0 Hz ; H'"1 : 4,67 ppm, J1'"-2'" : 8,0 Hz ; H""1 : 5,14 ppm, J1""-2"" : 3,5 Hz.

**EXEMPLE 7**

**Méthyl O-(3-désoxy-2,4,6-tri-O-sulfo-α-D-*ribo*-hexopyranosyl)-(1 → 4)-O-(3-désoxy-2-O-méthyl-β-D-*ribo*-hexopyranosyluronate)-(1 → 4)-O-(2,3,6-tri-O-sulfo-α-D-glucopyranosyl)-(1 → 4)-O-(3-O-méthyl-2-O-sulfo-α-L-idopyrano syluronate)-(1 → 4)-2,3,6-tri-O-sulfo-α-D-glucopyranoside dodecakis sel de sodium**

Ce composé a été préparé selon le procédé décrit dans l'Exemple 1, à partir du O-(6-O-acétyl-2,4-di-O-benzyl-3-désoxy-α-D-*ribo*-hexopyranosyl)-(1 → 4)-O-(benzyl-2-O-méthyl-3-désoxy-β-D-*ribo*-hexopyranosyluronate)-(1 → 4)-3,6-di-O-acétyl-2-O-benzyl-D-glucopyranosyl trichloroacétimidate, et du méthyl-2,3,6-tri-O-benzyl-4-O-(benzyl-2-O-benzyl-3-O-méthyl-α-L-idopyranosyluronate)-α-D- glucopyranoside. Ce dernier composé a été préparé selon la méthode décrite par M. Petitou et C.A.A. van Boeckel dans "Chemical synthesis of heparin fragments and analogues" pp. 203-210 - Progress in the Chemistry of Organic Natural Products, Ed. Springer Verlag Wien - N.Y. 1992.
$[\alpha]_D^{20}$ = + 46° (C = 0,58 dans $H_2O$)

Spectre de résonance magnétique nucléaire du proton : (500 MHz, solvant $D_2O$)

H1 : 5,10 ppm J1-2 : 3,0 Hz; H'1 : 5,10 ppm, J1'-2' : 5,0 Hz; H"1 : 5,48 ppm, J1"-2" : 3,60 Hz; H'"1 : 4,68 ppm, J1'"-2'" : 7,5 Hz; H""1 : 5,16 ppm, J1""-2"" : 3,6 Hz.

**EXEMPLE 8**

**Méthyl O-(3-désoxy-2,4-di-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-α-L-*lyxo*-hexopyranosyluronate)-(1 → 4)-2,3,6-tri-O-sulfo-β-D-glucopyranoside, hexadécakis sel de sodium**

Ce composé a été préparé à partir du O-benzyl-2-O-acétyl-3-désoxy-4-O-lévulinyl-β-D-*ribo*-hexopyranosyluronate)-(1 → 4)-3,6-di-O-acétyl-2-O-benzyl-D-glucopyranosyl) trichloroacétimidate (Préparation VI) et du 3-O-acétyl-1,6-anhydro-2-O-benzyl-4-O-(benzyl-2-O-acétyl-3-désoxy-β-D-*ribo*-hexopyranosyluro nate)-β-D-glucopyranose (Préparation V). En faisant réagir ces deux composés dans les conditions décrites dans l'Exemple 1, Stade A, on obtient le composé :

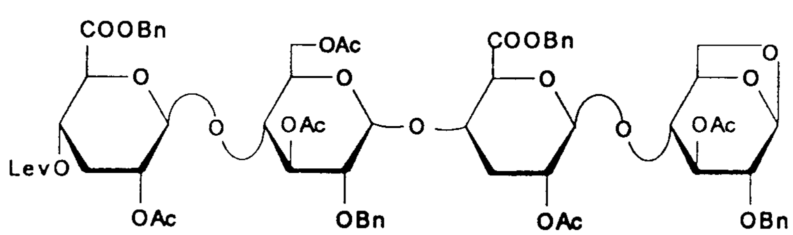

Rendement : 64 %.

Le produit ainsi obtenu est soumis à une acétolyse selon le procédé décrit dans la Préparation VI Stade A, puis à l'action de benzylamine dans les conditions décrites au Stade B de la Préparation VI, pour libérer l'hydroxyle anomère. L'imidate correspondant est obtenu ensuite par réaction avec le trichloroacétonitrile en présence de carbonate de potassium selon le procédé décrit dans la préparation VI Stade C.
En faisant réagir ce dernier produit avec le méthyl-3,6-di-O-acétyl-2-O-benzyl-4-O-(benzyl-2-O-acétyl-3-désoxy-α-L-*lyxo*-hexopyranosyluronate)-β-D-glucopyranoside (Préparation IX), on obtient le méthyl O-(3-désoxy-2,4-di-O-sulfo-β-D-*ribo*-hexopyranosyluronate)-(1 → 4)-O-(2,3,6-tri-O-sulfo-α-D-glucopyranosyl)(1 → 4)-O-(3-désoxy-2-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-α-L-*lyxo*-hexopyranosyluronate)-(1 → 4)-2,3,6-tri-O-sulfo-β-D-glucopyranoside, hexadécakis sel de sodium.

<u>Spectre de résonance magnétique nucléaire du proton : (500 MHz, solvant $D_2O$)</u>

H1 : 4,78 ppm J1-2 : 7,0 Hz ; H'1 : 5,22 ppm, J1'-2' : 1,9 Hz ; H"1 : 5,25 ppm, J1"-2" : 3,50 Hz ; H'"1 : 4,79 ppm, J1'"-2'" : 5,6 Hz ; H"" : 5,31 ppm, J1 -2 : 3,50 Hz ; H""'1 : 4,80 ppm, J1""'-2""' : 5,9 Hz.

## Revendications

**1.** Composés de formule I:

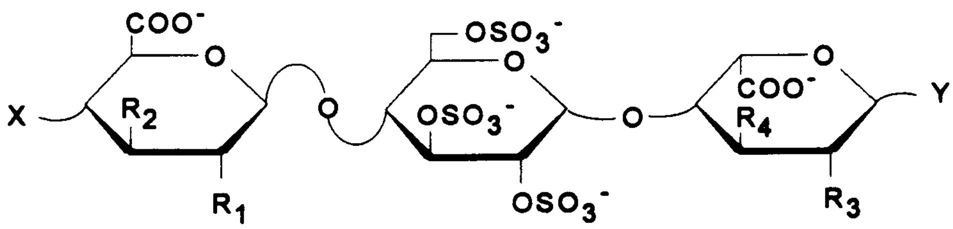

(I)

dans laquelle,

- X représente un radical $-OSO_3^-$, un radical de formule A,

R — O (A)

un radical de formule B,

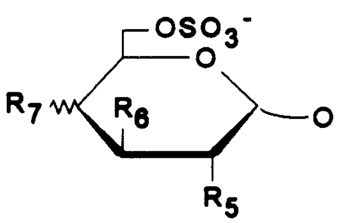

(B)

ou un radical de formule C,

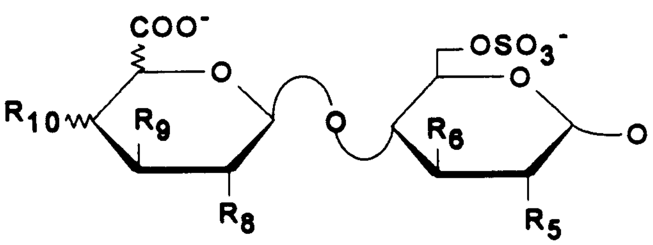

(C)

- Y représente un radical de formule D,

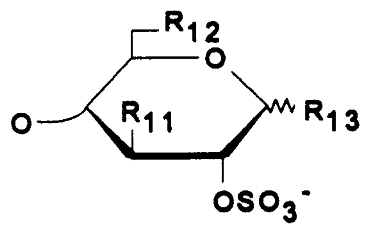

(D)

- R représente un radical alkyle, linéaire ou ramifié de 1 à 6 atomes de carbone,

- $R_1$, $R_3$, $R_5$, $R_7$, $R_8$, $R_{10}$, $R_{12}$ et $R_{13}$, identiques ou différents, représentent chacun un radical hydroxy, un radical alcoxy linéaire ou ramifié de 1 à 6 atomes de carbone, ou un radical $-OSO_3^-$,

- $R_2$, $R_4$, $R_6$, $R_9$ et $R_{11}$, identiques ou différents représentent chacun un atome d'hydrogène, un radical hydroxy, un radical alcoxy linéaire ou ramifié de 1 à 6 atomes de carbone, ou un radical $-OSO_3^-$,

à condition toutefois qu'au moins un des substituants présents $R_2$ ou $R_4$ ou $R_6$ ou $R_9$ ou $R_{11}$, représente un atome d'hydrogène,
sous forme de sels pharmaceutiquement acceptables et les acides correspondants.

2. Composés de formule I, selon la revendication 1, répondant à la formule Ia :

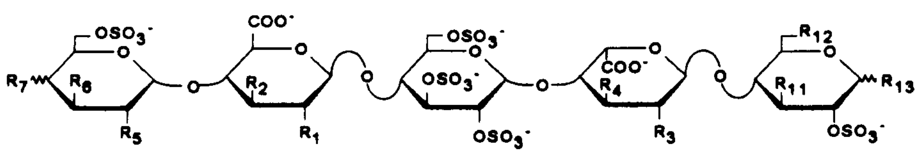

(Ia)

dans laquelle $R_1$, $R_2$, $R_3$, $R_4$, $R_5$, $R_6$, $R_7$, $R_{11}$, $R_{12}$, et $R_{13}$, ont la même signification que pour la formule I, selon la revendication 1.

3. Composés de formule I, selon la revendication 1, répondant à la formule Ib :

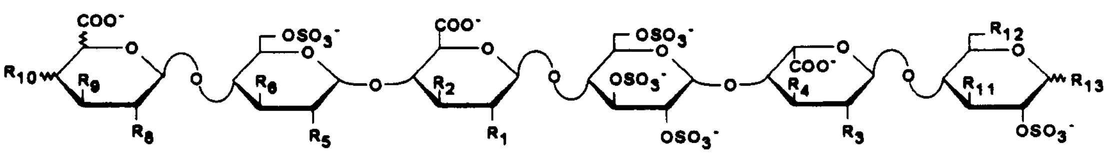

(Ib)

dans laquelle $R_1$, $R_2$, $R_3$, $R_4$, $R_5$, $R_6$, $R_8$, $R_9$, $R_{10}$, $R_{11}$, $R_{12}$, et $R_{13}$, ont la même signification que pour la formule I, selon la revendication 1.

4. Composés de formule I, selon la revendication 1, dans laquelle,

- $R_1$, $R_3$, $R_5$, $R_7$, $R_8$, $R_{10}$, et $R_{13}$ identiques ou différents, représentent chacun un radical alcoxy linéaire ou ramifié de 1 à 6 atomes de carbone, ou un radical $-OSO_3^-$,
- $R_2$, $R_4$, $R_6$ et $R_9$, identiques ou différents représentent chacun un atome d'hydrogène ou un radical alcoxy linéaire ou ramifié de 1 à 6 atomes de carbone,

- $R_{11}$ représente un atome d'hydrogène, un radical alcoxy linéaire ou ramifié de 1 à 6 atomes de carbone ou un radical $-OSO_3^-$,
  à condition toutefois qu'au moins un des substituants présents $R_2$ ou $R_4$ ou $R_6$ ou $R_9$ ou $R_{11}$ représente un atome d'hydrogène,
  et,
- $R_{12}$ représente un radical hydroxy ou un radical $-OSO_3^-$.

**5.** Composés de formule I, selon la revendication 1, dans laquelle $R_2$ représente un atome d'hydrogène.

**6.** Composés de formule I, selon la revendication 1, dans laquelle,

- $R_2$ et $R_6$ représentent un atome d'hydrogène,
- $R_3$, $R_{11}$ et $R_{12}$ représentent un radical $-OSO_3^-$,
  et,
- $R_{13}$ représente un radical alcoxy linéaire ou ramifié de 1 à 6 atomes de carbone.

**7.** Le méthyl O-(3-désoxy-2,4,6-tri-O-sulfo-α-D-*ribo*-hexopyranosyl)-(1 → 4)-O-(3-désoxy-2-O-sulfo-β-D-*ribo*-hexopyranosyluronate)-(1 → 4)-O-(2,3,6-tri-O-sulfo -α-D-glucopyranosyl)-(1 → 4)-O-(3-désoxy-2-O-sulfo-α-L-*lyxo*-hexopyranosyluronate)-(1 → 4)-2,3,6-tri-O-sulfo-β-D-glucopyranoside, composé de formule I, selon la revendication 1 et ses sels avec une base, pharmaceutiquement acceptables.

**8.** Le méthyl O-(3-désoxy-2,4-di-O-méthyl-6-O-sulfo-α-D-*ribo*-hexopyranosyl)-(1 → 4)-O-(3-désoxy-2-O-sulfo-β-D-*ribo*-hexopyranosyluronate)-(1 → 4)-O-(2,3,6-tri -O-sulfo-α-D-glucopyranosyl)-(1 → 4)-O-(3-désoxy-2-O-sulfo-α-L-*lyxo*-hexopyranosyluronate)-(1 → 4)-2,3,6-tri-O-sulfo-β-D-glucopyranoside, composé de formule I, selon la revendication 1 et ses sels avec une base, pharmaceutiquement acceptables.

**9.** Le méthyl O-(3-désoxy-4-O-méthyl-2,6-di-O-sulfo-α-D-*ribo*-hexopyranosyl)-(1 → 4)-O-(3-désoxy-2-O-sulfo-β-D-*ribo*-hexopyranosyluronate)-(1 → 4)-O-(2,3,6-tri-O-sulfo-α-D-glucopyranosyl)-(1 → 4)-O-(3-désoxy-2-O-sulfo-α-L-*lyxo*-hexo-pyranosyluronate)-(1 → 4)-2,3,6-tri-O-sulfo-β-D-glucopyranoside, composé de formule I, selon la revendication 1 et ses sels avec une base, pharmaceutiquement acceptables.

**10.** Procédé de préparation des composés de formule I, selon la revendication 1, caractérisé en ce que l'on fait réagir un composé de formule II :

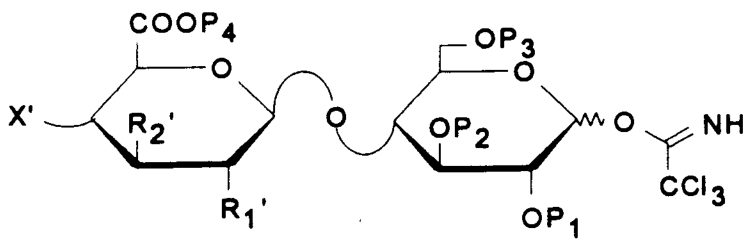

(II)

dans laquelle,

- X' représente un radical chloroacétyloxy, un radical lévulinyloxy, un radical de formule A selon la formule I, de la revendication 1,
  un radical de formule $B_1$,

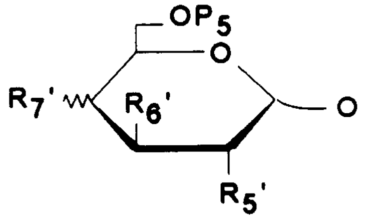

$(B_1)$

ou un radical de formule $C_1$,

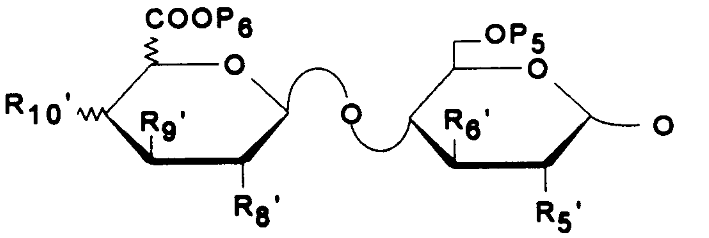

$(C_1)$

- $P_1$, $P_2$, $P_3$ et $P_5$, identiques ou différents, représentent chacun un groupe protecteur tel qu'un radical acyle acyclique de 1 à 6 atomes de carbone, de préférence un radical acétyle, un radical acyle aromatique, de préférence un radical benzoyle, un radical alc-2-ényle de 2 à 7 atomes de carbone, de préférence un radical allyle, ou un radical benzyle,
- $P_4$, $P_6$, identiques ou différents représentent chacun un groupe protecteur tel qu'un radical alkyle de 1 à 6 atomes de carbone de préférence un radical méthyle ou, un radical benzyle,
- $R_1'$, $R_5'$, $R_8'$, identiques ou différents, représentent chacun un radical alcoxy linéaire ou ramifié de 1 à 6 atomes de carbone, un radical acyloxy acyclique de 1 à 6 atomes de carbone, de préférence un radical acétoxy, un radical acyloxy aromatique, de préférence un radical benzoyloxy, ou un radical alc-2-ényloxy de 2 à 7 atomes de carbone, de préférence un radical allyloxy,
- $R_7'$, $R_{10}'$, identiques ou différents, ont les significations données pour $R_1'$ ou ils représentent un radical chloroacétoxy ou un radical lévulinyloxy,
  et,
- $R_2'$, $R_6'$ et $R_9'$, identiques ou différents, représentent chacun un atome d'hydrogène, ou ont les significations données pour $R_1'$,

avec un composé de formule III :

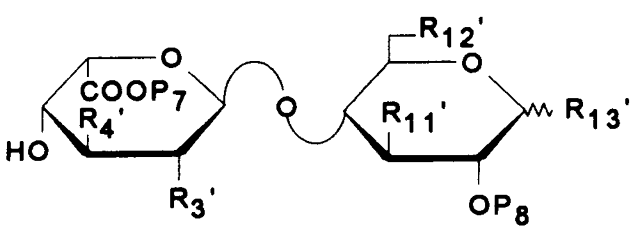

(III)

dans laquelle,

- $P_7$ a les significations données pour $P_4$ dans la formule II,
- $P_8$ a les significations données pour $P_1$ dans la formule II,
- $R_4'$ et $R_{11}'$ identiques ou différents, ont les mêmes significations que $R_2'$ dans la formule II,
  et,
- $R_3'$, $R_{12}'$ et $R_{13}'$ identiques ou différents ont les significations données pour $R_1'$ dans la formule II,

pour préparer les composés de formule IV :

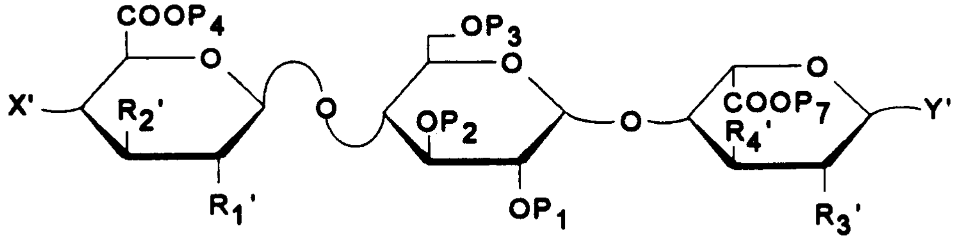

(IV)

dans laquelle,

- X', $P_1$, $P_2$, $P_3$, $P_4$, $R_1$' et $R_2$' ont la même signification que pour la formule II,
- $P_7$, $R_3$' et $R_4$' ont la même signification que pour la formule III, et,
- Y' représente un radical de formule $D_1$ :

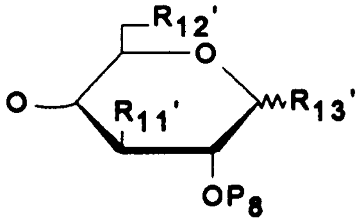

($D_1$)

dans laquelle,

- $P_8$, $R_{11}$', $R_{12}$' et $R_{13}$' ont la même signification que pour la formule III,

que l'on soumet ensuite soit à une hydrogénation catalytique puis à une saponification et à une sulfatation, soit d'abord à une saponification, puis à une sulfatation et ensuite à une hydrogénation catalytique, soit d'abord à une hydrogénation catalytique, puis à une sulfatation et ensuite à une saponification, pour obtenir les composés de formule I.

**11.** Procédé de préparation des composés de formule I, selon la revendication 1, caractérisé en ce que l'on fait réagir un monosaccharide comportant des groupes protecteurs sur les radicaux hydroxy et éventuellement sur les radicaux carboxy s'il en dispose, avec un autre monosaccharide protégé, pour former un disaccharide sur lequel ensuite, on fait réagir un autre monosaccharide protégé, pour former un trisaccharide protégé à partir duquel, on peut obtenir un tetrasaccharide protégé puis un pentasaccharide protégé et ensuite, un hexasaccharide protégé, lesquels tetra, penta ou hexasaccharides sont ensuite déprotégés et éventuellement sulfatés ou, d'abord partiellement déprotégés, puis sulfatés, et ensuite déprotégés.

**12.** Composés de formule I selon l'une quelconque des revendications 1 à 9 pour utilisation comme médicament.

**13.** Compositions pharmaceutiques contenant comme principe actif un composé selon l'une quelconque des revendications 1 à 9, ou un de ses sels avec une base pharmaceutiquement acceptable, en association ou en mélange avec un excipient inerte, non toxique, pharmaceutiquement acceptable.

**14.** Le 1,6:2,3 di-anhydro-4-O-(2,4,6-tri-O-benzoyl-3-désoxy-β-D-*ribo*-hexopyranosyl) -β-D-mannopyranose, composé de formule IX :

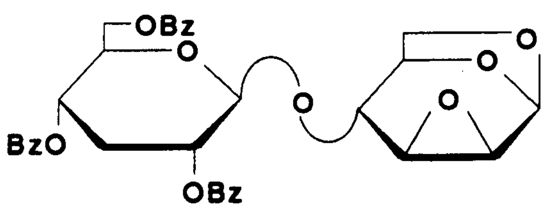

(IX)

dans laquelle Bz représente un radical benzoyle.

**15.** Le 1,6:2,3-di-anhydro-4-O-(3-désoxy-β-D-*ribo*-hexopyranosyl)-β-D-mannopyranose, composé de formule X :

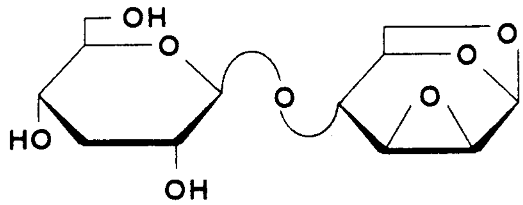

(X)

**16.** Composés de formule XI :

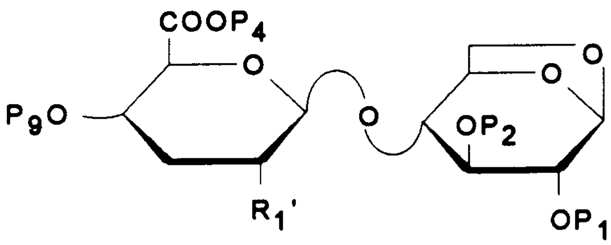

(XI)

dans laquelle,

- $R_1$' représente un radical alcoxy linéaire ou ramifié de 1 à 6 atomes de carbone, un radical acyloxy acyclique de 1 à 6 atomes de carbone, de préférence un radical acétoxy, un radical acyloxy aromatique, de préférence un radical benzoyloxy, ou un radical alc-2-ényloxy de 2 à 7 atomes de carbone, de préférence un radical allyloxy,
- $P_1$, $P_2$, identiques ou différents, représentent chacun un groupe protecteur choisi parmi un radical acyle acyclique de 1 à 6 atomes de carbone, de préférence un radical acétyle, un radical acyle aromatique, de préférence un radical benzoyle, un radical alc-2-ényle de 2 à 7 atomes de carbone, de préférence un radical allyle ou un radical benzyle,
- $P_4$, représente un groupe protecteur choisi parmi un radical alkyle de 1 à 6 atomes de carbone de préférence un radical méthyle ou un radical benzyle,
  et,
- $P_9$ représente un radical lévulinyle ou un radical chloroacétyle.

## Claims

**1.** Compounds of formula I

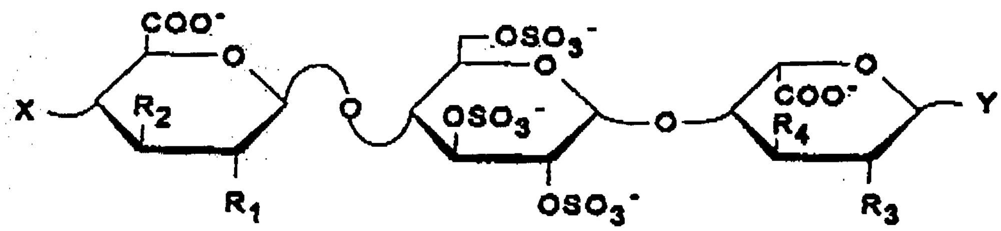

(I)

wherein

- X denotes a radical $-OSO_3^-$, a radical of formula A,

RO (A)

a radical of formula B,

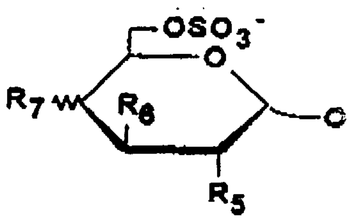

(B)

or a radical of formula C,

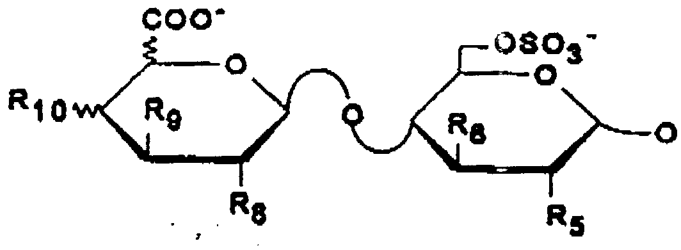

(C)

- Y represents a radical of formula D,

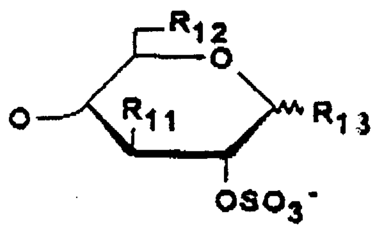

(D)

- R denotes a straight-chained or- branched $C_{1-6}$-alkyl radical,
- $R_1$, $R_3$, $R_5$, $R_7$, $R_8$, $R_{10}$, $R_{12}$ and $R_{13}$, which may be identical or different, each represent a hydroxy radical, a straight-chained or branched $C_{1-6}$-alkoxy radical or a radical $-OSO_3^-$,
- $R_3$, $R_4$, $R_6$, $R_9$ and $R_{11}$, which may be identical or different, each denote a hydrogen atom, a hydroxy radical, a straight-chained or branched $C_{1-6}$-alkoxy radical or a radical $-OSO_3^-$,

with the proviso that at least one of the substituents present, $R_2$ or $R_4$ or $R_6$ or $R_9$ or $R_{11}$, represents a hydrogen atom,

in the form of pharmaceutically acceptable salts and the corresponding acids.

**2.** Compounds of formula I according to claim 1, corresponding to formula Ia:

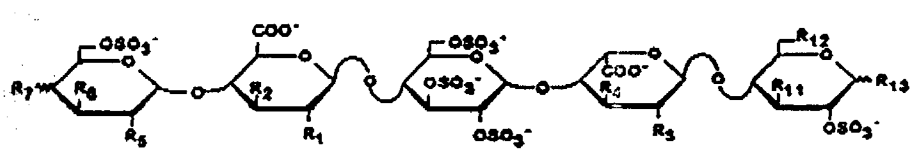

(Ia)

wherein $R_1$, $R_2$, $R_3$, $R_4$, $R_5$, $R_6$, $R_7$, $R_{11}$, $R_{12}$ and $R_{13}$ have the same meanings as in formula I, according to claim 1.

**3.** Compounds of formula I according to claim 1, corresponding to formula Ib:

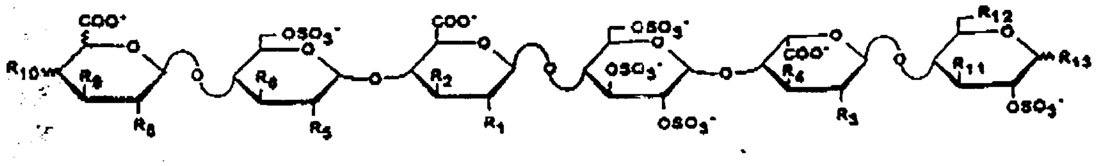

(Ib)

wherein $R_1$, $R_2$, $R_3$, $R_4$, $R_5$, $R_6$, $R_8$, $R_9$, $R_{10}$, $R_{11}$, $R_{12}$ and $R_{13}$ have the same meaning as in formula I, according to claim 1.

**4.** Compounds of formula I according to claim 1, wherein

- $R_1$, $R_3$, $R_5$, $R_7$, $R_8$, $R_{10}$ and $R_{13}$, which may be identical or different, each represent a straight-chained or branched $C_{1-6}$-alkoxy group or a radical $-OSO_3^-$,
- $R_2$, $R_4$, $R_6$ and $R_9$, which may be identical or different, each denote a hydrogen atom or a straight-chained or branched $C_{1-6}$-alkoxy radical,
- $R_{11}$ denotes a hydrogen atom, a straight-chained or branched $C_{1-6}$-alkoxy radical or a radical $-OSO_3^-$, with the proviso that at least one of the substituents present, $R_2$ or $R_4$ or $R_6$ or $R_9$ or $R_{11}$, denotes a hydrogen atom, and
- $R_{12}$ denotes a hydroxy radical or a radical $-OSO_3^-$.

**5.** Compounds of formula I according to claim 1 wherein $R_2$ denotes a hydrogen atom.

**6.** Compounds of formula I according to claim 1, wherein

- $R_2$ and $R_6$ denote a hydrogen atom,
- $R_3$, $R_{11}$ and $R_{12}$ denote a radical $-OSO_3^-$, and
- $R_{13}$ denotes a straight-chained or branched $C_{1-6}$-alkoxy radical.

**7.** Methyl O-(3-deoxy-2,4,6-tri-sulfo-α-D-*ribo*-hexaopyranosyl)-(1→4)-O-(3-deoxy-2-O-sulfo-β-D-*ribo*-hexopyranosyluronate)-(1→4)-O-(2,3,6-tri-O-sulfo-α-D-glucopyranosyi)-(1→4)-O-(3-deoxy-2-O-sulfo-α-L-*lyxo*-hexopyranosyluronate)-(1→4)-2,3,6-tri-O-sulfo-β-D-glucopyranoside, compound of formula I, according to claim 1 and the pharmaceutically acceptable salts thereof with a base.

**8.** Methyl O-(3-deoxy-2,4-di-O-methyl-6-O-sulfo-α-D-*ribo*-hexaopyranosyl)-(1→4)-O-(3-deoxy-2-O-sulfo-β-D-*ribo*-hexopyranosyluronate)-(1→4)-O-(2,3,6-tri-O-sulfo-α-D-glucopyranosyl)-(1→4)-O-(3-deoxy-2-O-sulfo-α-L-*lyxo*-hexopyranosyl-uronate)-(1→4)-2,3,6-tri-O-sulfo-β-D-glucopyranoside, compound of formula I, according to claim 1 and the pharmaceutically acceptable salts thereof with a base.

**9.** Methyl O-(3-deoxy-4-O-methyl-2,6-di-O-sulfo-α-D-*ribo*-hexaopyranosyl)-(1→4)-O-(3-deoxy-2-O-sulfo-β-D-*ribo*-hexopyranosyluronate)-(1→4)-O-(2,3,6-tri-O-sulfo-α-D-glucopyranosyl)-(1→4)-O-(3-deoxy-2-O-sulfo-α-L-*lyxo*-hexopyranosyl-uronate)-(1→4)-2,3,6-tri-O-sulfo-β-D-glucopyranoside, compound of formula I, according to claim 1 and the pharmaceutically acceptable salts thereof with a base.

**10.** Process for preparing compounds of formula I according to claim 1, characterised in that a compound of formula II

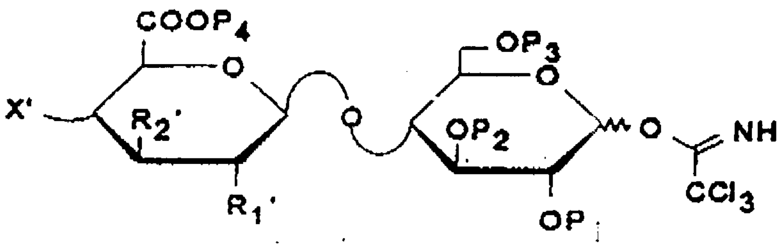

(II)

wherein

- X' represents a chloroacetyloxy radical, a levulinyloxy radical, a radical of formula A according to formula I in claim 1,
  a radical of formula $B_1$,

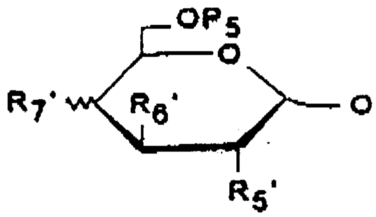

($B_1$)

or a radical of formula $C_1$,

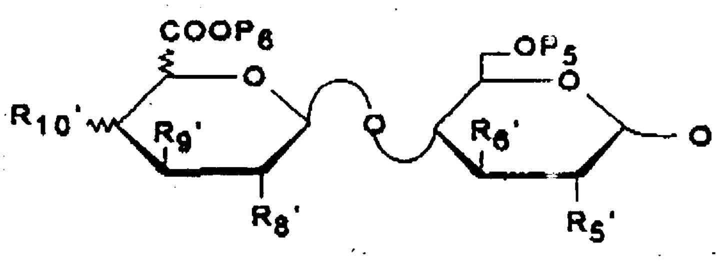

($C_1$)

- $P_1$, $P_2$, $P_3$ and $P_5$, which may be identical or different, each denote a protecting group such as an acyclic $C_{1-6}$-acyl radical, preferably an acetyl radical, an aromatic acyl radical, preferably a benzoyl radical, a $C_{2-7}$-alc-2-enyl radical, preferably an allyl radical, or a benzyl radical,
- $P_4$ and $P_6$ which may be identical or different, each denotes a protecting group such as a $C_{1-6}$-alkyl radical, preferably a methyl radical or a benzyl radical,
- $R_1$', $R_5$', and $R_8$' which may be identical or different, each denote a straight-chained or branched $C_{1-6}$-alkoxy radical, an acyclic $C_{1-6}$-acyloxy radical, preferably an acetoxy radical, an aromatic acyloxy radical, preferably a benzoyloxy radical or a $C_{2-7}$-alc-2-enyloxy radical, preferably an allyloxy radical,
- $R_7$' and $R_{10}$' which may be identical or different, have the meanings given for $R_1$' or they represent a chloroacetoxy radical or a levulinyloxy radical, and
- $R_2$', $R_6$' and $R_9$' which may be identical or different, each represent a hydrogen atom, or have the meanings given for $R_1$',

is reacted with a compound of formula III:

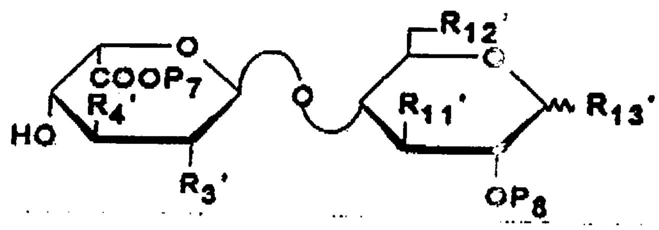

(III)

wherein

- $P_7$ has the meanings given for $P_4$ in formula II,
- $P_8$ has the meanings given for $P_1$ in formula II,
- $R_4$' and $R_{11}$', which may be identical or different, have the same meanings as $R_2$' in formula II and
- $R_3$', $R_{12}$' and $R_{13}$', which may be identical or different, have the meanings given for $R_1$' in formula II,

in order to prepare the compounds of formula IV:

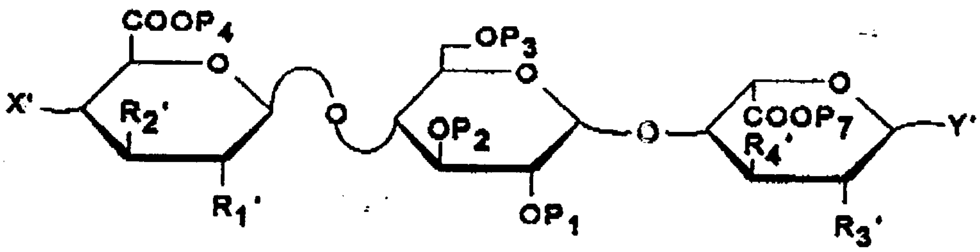

(IV) :

wherein

- $X'_1$, $P_1$, $P_2$, $P_3$, $P_4$, $R_1$' and $R_2$' have the same meanings as in formula II,
- $P_7$, $R_3$' and $R_4$' have the same meanings as in formula III, and
- Y' denotes a radical of formula $D_1$:

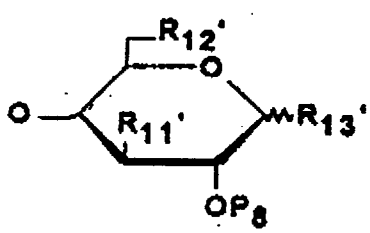

($D_1$)

wherein

- $P_8$, $R_{11}$', $R_{12}$' and $R_{13}$' have the same meanings as in formula III,

which is then subjected to catalytic hydrogenation followed by saponification and sulfatation, or first to saponification and then to sulfatation and finally to catalytic hydrogenation, or first to catalytic hydrogenation then sulfatation and finally saponification, to obtain the compounds of formula I.

**11.** Process for preparing the compounds of formula I according to claim 1, characterised in that a monosaccharide containing protecting groups on the hydroxy radical and possibly on the carboxyl radicals if it has any, is reacted with another protected monosaccharide, to form a disaccharide with which another protected monosaccharide is subsequently reacted, to form a protected trisaccharide, from which a protected tetrasaccharide can be obtained followed by a protected pentasaccharide and, subsequently, a protected hexasaccharide, the said tetra-, penta- or

hexasaccharides subsequently being deprotected and possibly sulfated or first of all partly deprotected, then sulfated and then deprotected.

12. Compounds of formula I according to any one of claims 1 to 9 for use as a medicament.

13. Pharmaceutical compositions containing as active principle a compound according to any one of claims 1 to 9, or one of the salts thereof with a pharmaceutically acceptable base, combined or admixed with an inert, non toxic, pharmaceutically acceptable excipient.

14. 1,6;2,3 di-anhydro-4-O-(2,4,6-tri-O-benzoyl-3-deoxy-β-D-*ribo*-hexopyranosyl)-β-D-mannopyranose, compound of formula IX:

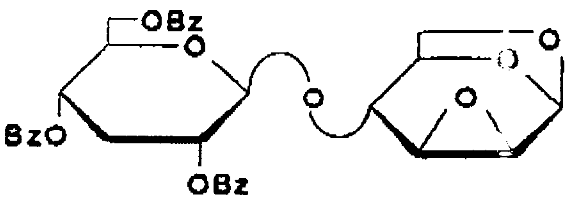

(IX)

wherein Bz denotes a benzoyl radical.

15. 1,6:2,3 di-anhydro-4-O-(3-deoxy-β-D-*ribo*-hexopyranosyl)-β-D-mannopyranose, compound of formula X:

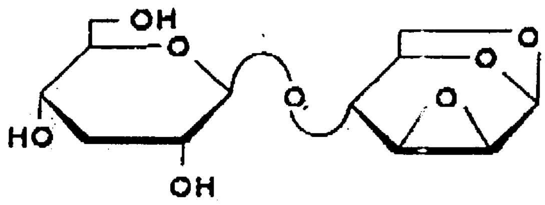

(X)

16. Compounds of formula XI:

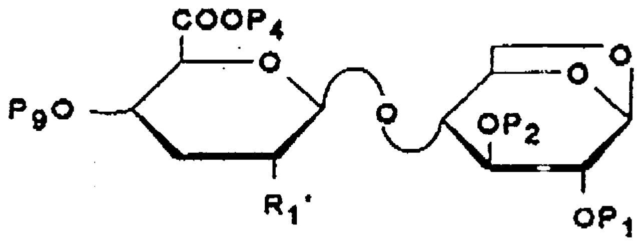

(XI)

wherein

- $R_1$' denotes a straight-chained or branched $C_{1-6}$-alkoxy radical, a $C_{1-6}$-acylic acyloxy radical, preferably an acetoxy radical, an aromatic acyloxy radical, preferably a benzoyloxy radical or a $C_{2-7}$-alc-2-enyloxy radical, preferably an allyloxy radical,
- $P_1$ and $P_2$ which may be identical or different, each denote a protecting group selected from an acyclic $C_{1-6}$-acyl radical, preferably an acetyl radical, an aromatic acyl radical, preferably a benzoyl radical, a $C_{2-7}$-alc-2-enyl radical, preferably an allyl radical or a benzyl radical,
- $P_4$ denotes a protecting group selected from a $C_{1-6}$-alkyl radical, preferably a methyl radical or a benzyl radical, and
- $P_9$ denotes a levulinyl radical or a chloroacetyl radical.

## Patentansprüche

1. Verbindungen der Formel I:

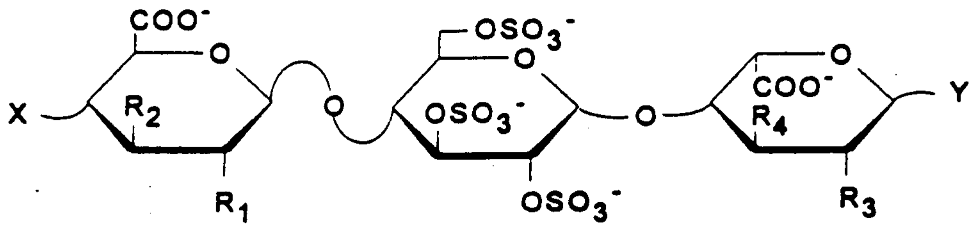

(I)

in der

- X eine Gruppe $-OSO_3^-$, eine Gruppe der Formel A

$$R - O \qquad (A)$$

eine Gruppe der Formel B

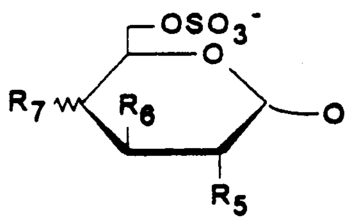

(B)

oder eine Gruppe der Formel C

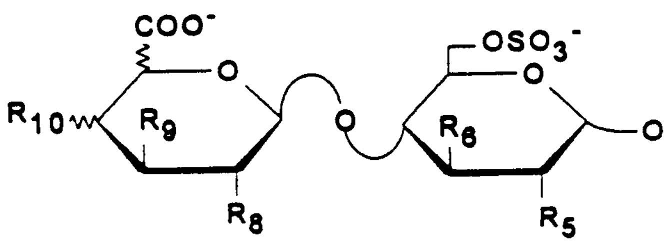

(C)

- Y eine Gruppe der Formel D

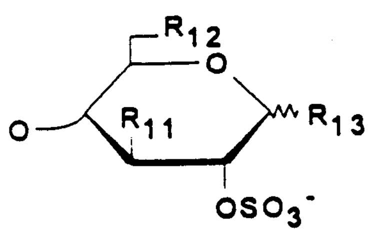

(D)

- R eine geradkettige oder verzweigte Alkylgruppe mit 1 bis 6 Kohlenstoffatomen,

- $R_1$, $R_3$, $R_5$, $R_7$, $R_8$, $R_{10}$, $R_{12}$ und $R_{13}$, die gleichartig oder verschieden sein können, jeweils eine Hydroxylgruppe, eine geradkettige oder verzweigte Alkoxygruppe mit 1 bis 6 Kohlenstoffatomen oder eine Gruppe -$OSO_3^-$, und

- $R_2$, $R_4$, $R_6$, $R_9$ und $R_{11}$, die gleichartig oder verschieden sein können, jeweils ein Wasserstoffatom, eine Hydroxylgruppe, eine geradkettige oder verzweigte Alkoxygruppe mit 1 bis 6 Kohlenstoffatomen oder eine Gruppe -$OSO_3^-$

mit der Maßgabe bedeuten, daß mindestens einer der vorhandenen Substituenten $R_2$ oder $R_4$ oder $R_6$ oder $R_9$ oder $R_{11}$ ein Wasserstoffatom darstellt, in Form der pharmazeutisch annehmbaren Salze und der entsprechenden Säuren.

**2.** Verbindungen der Formel I nach Anspruch 1 der Formel Ia:

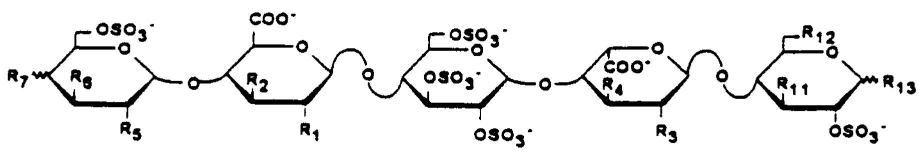

(Ia)

in der $R_1$, $R_2$, $R_3$, $R_4$, $R_5$, $R_6$, $R_7$, $R_{11}$, $R_{12}$ und $R_{13}$ die bezüglich der Formel I in Anspruch 1 angegebenen Bedeutungen besitzen.

**3.** Verbindungen der Formel I nach Anspruch 1 der Formel Ib:

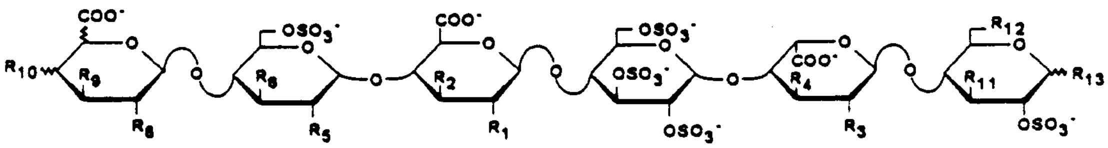

(Ib)

in der $R_1$, $R_2$, $R_3$, $R_4$, $R_5$, $R_6$, $R_8$, $R_9$, $R_{10}$, $R_{11}$, $R_{12}$ und $R_{13}$ die bezüglich der Formel I in Anspruch 1 angegebenen Bedeutungen besitzen.

**4.** Verbindungen der Formel I nach Anspruch 1, in der

- $R_1$, $R_3$, $R_5$, $R_7$, $R_8$, $R_{10}$ und $R_{13}$, die gleichartig oder verschieden sein können, jeweils eine geradkettige oder verzweigte Alkoxygruppe mit 1 bis 6 Kohlenstoffatomen oder eine Gruppe -$OSO_3^-$,
- $R_2$, $R_4$, $R_6$ und $R_9$, die gleichartig oder verschieden sein können, jeweils ein Wasserstoffatom oder eine geradkettige oder verzweigte Alkoxygruppe mit 1 bis 6 Kohlenstoffatomen,
- $R_{11}$ ein Wasserstoffatom, eine geradkettige oder verzweigte Alkoxygruppe mit 1 bis 6 Kohlenstoffatomen oder eine Gruppe -$OSO_3^-$.
  mit der Maßgabe, daß mindestens einer der vorhandenen Substituenten $R_2$ oder $R_4$ oder $R_6$ oder $R_9$ oder $R_{11}$ ein Wasserstoffatom darstellt, und
- $R_{12}$ eine Hydroxylgruppe oder eine Gruppe -$OSO_3^-$ bedeuten.

**5.** Verbindungen der Formel I nach Anspruch 1, worin $R_2$ ein Wasserstoffatom darstellt.

**6.** Verbindungen der Formel I nach Anspruch 1, in der

- $R_2$ und $R_6$ ein Wasserstoffatom.
- $R_3$, $R_{11}$ und $R_{12}$ eine Gruppe -$OSO_3^-$ und
- $R_{13}$ eine geradkettige oder verzweigte Alkoxygruppe mit 1 bis 6 Kohlenstoffatomen bedeuten.

**7.** Verbindung nach Anspruch 1, nämlich Methyl-O-(3-desoxy-2,4,6-tri-O-sulfo-α-D-*ribo*-hexopyranosyl)-(1→4)-O-(3-desoxy-2-O-sulfo-β-D-*ribo*-hexopyranosyluronat)-(1→4)-O-(2,3,6-tri-O-sulfo-α-D-glucopyranosyl)-(1→4)-O-(3-desoxy-2-O-sulfo-α-L-*lyxo*-hexopyranosyluronat)-(1→4)-2,3,6-tri-O-sulfo-β-D-glucopyranosid und dessen Salze mit einer pharmazeutisch annehmbaren Base.

8. Verbindung nach Anspruch 1, nämlich Methyl-O-(3-desoxy-2,4-di-O-methyl-6-O-sulfo-α-D-*ribo*-hexopyranosyl)-(1→4)-O-(3-desoxy-2-O-sulfo-β-D-*ribo*-hexopyranosyluronat)-(1→4)-O-(2,3,6-tri-O-sulfo-α-D-glucopyranosyl)-(1→4)-O-(3-desoxy-2-O-sulfo-α-L-*lyxo*-hexopyranosyluronat)-(1→4)-2,3,6-tri-O-sulfo-β-D-glucopyranosid und dessen Salze mit einer pharmazeutisch annehmbaren Base.

9. Verbindung nach Anspruch 1, nämlich Methyl-O-(3-desoxy-4-O-methyl-2,6-di-O-sulfo-α-D-*ribo*-hexopyranosyl)-(1→4)-O-(3-desoxy-2-O-sulfo-β-D-*ribo*-hexopyranosyluronat)-(1→4)-O-(2,3,6-tri-O-sulfo-α-D-glucopyranosyl)-(1→4)-O-(3-desoxy-2-O-sulfo-α-L-*lyxo*-hexopyranosyluronat)-(1→4)-2,3,6-tri-O-sulio-ß-D-glucopyranosid und dessen Salze mit einer pharmazeutisch annehmbaren Base.

10. Verfahren zur Herstellung der Verbindungen der Formel I nach Anspruch 1, dadurch gekennzeichnet, daß man eine Verbindung der Formel II:

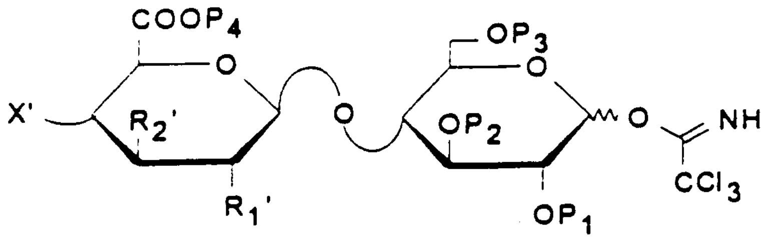

(II)

in der

X' eine Chloracetyloxygruppe, eine Laevulinyloxygruppe, eine Gruppe A entsprechend der Formel I von Anspruch 1,

eine Gruppe der Formel $B_1$:

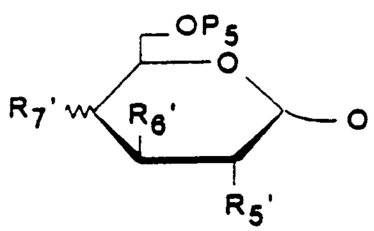

($B_1$)

oder eine Gruppe der Formel $C_1$:

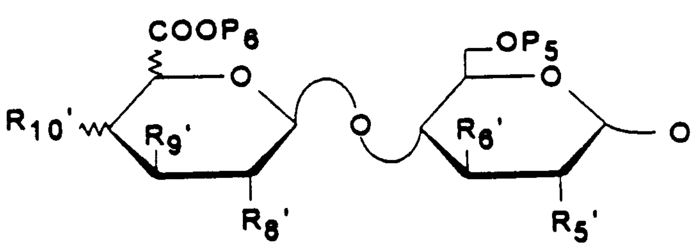

($C_1$)

- $P_1$, $P_2$, $P_3$ und $P_5$, die gleichartig oder verschieden sind, jeweils eine Schutzgruppe, wie eine acyclische Acylgruppe mit 1 bis 6 Kohlenstoffatomen, vorzugsweise eine Acetylgruppe, eine aromatische Acylgruppe, vorzugsweise eine Benzoylgruppe, eine Alk-2-enylgruppe mit 2 bis 7 Kohlenstoffatomen, vorzugsweise eine Allylgruppe, oder eine Benzylgruppe,
- $P_4$ und $P_6$, die gleichartig oder verschieden sein können, jeweils eine Schutzgruppe, wie eine Alkylgruppe mit 1 bis 6 Kohlenstoffatomen, vorzugsweise eine Methylgruppe, oder eine Benzylgruppe,
- $R_1$', $R_5$' und $R_8$', die gleichartig oder verschieden sein können, jeweils eine geradkettige oder verzweigte Alkoxygruppe mit 1 bis 6 Kohlenstoffatomen, eine acyclische Acyloxygruppe mit 1 bis 6 Kohlenstoffatomen, vorzugsweise eine Acetoxygruppe, eine aromatische Acyloxygruppe, vorzugsweise eine Benzoyloxygruppe, oder eine Alk-2-enyloxygruppe mit 2 bis 7 Kohlenstoffatomen, vorzugsweise eine Allyloxygruppe,

- $R_7$' und $R_{10}$', die gleichartig oder verschieden sein können, die für $R_1$' angegebenen Bedeutungen besitzen oder eine Chloracetoxygruppe oder eine Laevulinyloxygruppe und
- $R_2$', $R_6$' und $R_9$', die gleichartig oder verschieden sein können, jeweils die für $R_1$' angegebenen Bedeutungen besitzen oder ein Wasserstoffatom bedeuten,

mit einer Verbindung der Formel III:

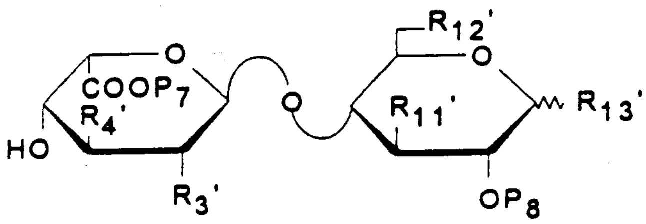

(III)

in der

- $P_7$ die für $P_4$ bezüglich der Formel II angegebenen Bedeutungen besitzt,
- $P_8$ die für $P_1$ bezüglich der Formel II angegebenen Bedeutungen besitzt,
- $R_4$' und $R_{11}$', die gleichartig oder verschieden sein können, die gleichen Bedeutungen besitzen, wie sie für $R_2$' bezüglich der Formel II angegeben sind, und
- $R_3$', $R_{12}$' und $R_{13}$', die gleichartig oder verschieden sein können, die bezüglich der Formel II angegebenen Bedeutungen von $R_1$' besitzen, umsetzt

zur Bildung der Verbindungen der Formel IV:

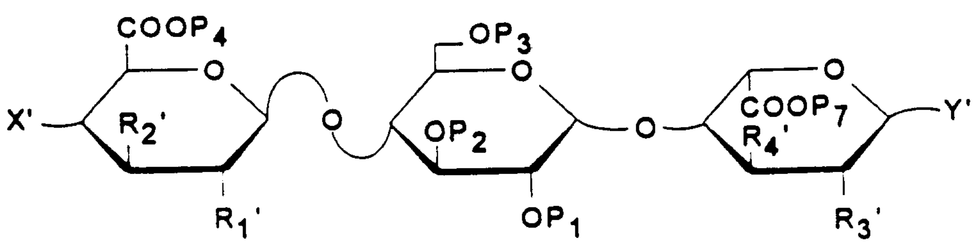

(IV)

in der

- X', $P_1$, $P_2$, $P_3$, $P_4$, $R_1$' und $R_2$' die bezüglich der Formel II angegebenen Bedeutungen besitzen,
- $P_7$, $R_3$' und $R_4$' die bezüglich der Formel III angegebenen Bedeutungen besitzen und
- Y' eine Gruppe der Formel $D_1$ bedeutet:

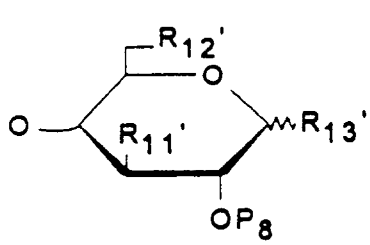

($D_1$)

in der

- $P_8$, $R_{11}$', $R_{12}$' und $R_{13}$' die bezüglich der Formel III angegebenen Bedeutungen besitzen,

welche man anschließend entweder einer katalytischen Hydrierung und dann einer Verseifung und einer Sulfatierung oder zunächst einer Verseifung, dann einer Sulfatierung und anschließend einer katalytischen Hydrierung oder zunächst einer katalytischen Hydrierung, dann einer Sulfatierung und anschließend einer Verseifung unterwirft zur Bildung der Verbindungen der Formel I.

**11.** Verfahren zur Herstellung der Verbindungen der Formel I nach Anspruch 1, dadurch gekennzeichnet, daß man ein Monosaccharid, welches Schutzgruppen an den Hydroxylgruppen und gegebenenfalls an den gegebenenfalls vorhandenen Carboxygruppen trägt, mit einem weiteren geschützten Monosaccharid umsetzt zur Bildung eines Disaccharids, welches man anschließend mit einem weiteren geschützten Monosaccharid umsetzt zur Bildung eines geschützten Trisaccharids ausgehend von welchem man ein geschütztes Tetrasaccharid und dann ein geschütztes Pentasaccharid und schließlich ein geschütztes Hexasaccharid erhält, welche Tetra-, Penta- oder Hexasaccharide man anschließend von ihren Schutzgruppen befreit und gegebenenfalls sulfatiert oder zunächst teilweise von ihren Schutzgruppen befreit, dann sulfatiert und anschließend von den Schutzgruppen befreit.

**12.** Verbindungen der Formel I nach irgendeinem der Ansprüche 1 bis 9 zur Verwendung als Arzneimittel.

**13.** Pharmazeutische Zubereitungen enthaltend als Wirkstoff eine Verbindung nach irgendeinem der Ansprüche 1 bis 9 oder eines ihrer Salze mit einer pharmazeutisch annehmbaren Base in Gegenwart oder in Mischung mit einem inerten, nichttoxischen, pharmazeutisch annehmbaren Trägermaterial.

**14.** 1,6:2,3-Di-anhydro-4-O-(2,4,6-tri-O-benzoyl-3-desoxy-β-D-*ribo*-hexopyranosyl)-β-D-mannopyranose der Formel IX:

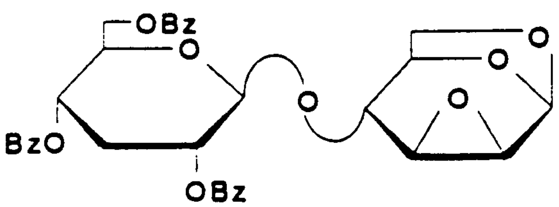

(IX)

in der Bz eine Benzoylgruppe bedeutet.

**15.** 1,6:2,3-Di-anhydro-4-O-(3-desoxy-β-D-*ribo*-hexopyranosyl)-β-D-mannopyranose der Formel X:

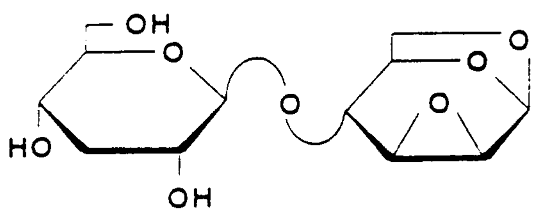

(X)

**16.** Verbindungen der Formel XI:

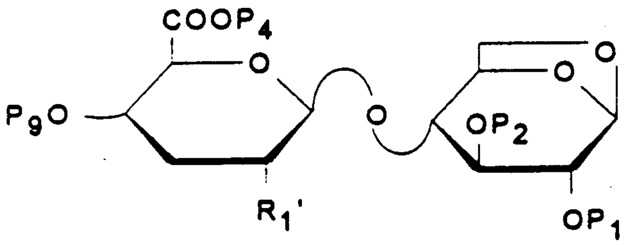

(XI)

in der

- $R_1'$ eine geradkettige oder verzweigte Alkoxygruppe mit 1 bis 6 Kohlenstoffatomen, eine acyclische Acyloxygruppe mit 1 bis 6 Kohlenstoffatomen, vorzugsweise eine Acetoxygruppe, eine aromatische Acyloxygruppe, vorzugsweise eine Benzoyloxygruppe, eine Alk-2-enyloxygruppe mit 2 bis 7 Kohlenstoffatomen, vorzugsweise eine Alkyloxygruppe,
- $P_1$ und $P_2$, die gleichartig oder verschieden sein können, jeweils eine Schutzgruppe ausgewählt aus einer acyclischen Acylgruppe mit 1 bis 6 Kohlenstoffatomen, vorzugsweise eine Acetylgruppe, eine aromatische Acylgruppe, vorzugsweise eine Benzoylgruppe, eine Alk-2-enylgruppe mit 2 bis 7 Kohlenstoffatomen, vorzugsweise eine Allylgruppe, oder eine Benzylgruppe,
- $P_4$ eine Schutzgruppe ausgewählt aus einer Alkylgruppe mit 1 bis 6 Kohlenstoffatomen, vorzugsweise eine Methylgruppe, oder eine Benzylgruppe,
  und
- $P_9$ eine Laevulinylgruppe oder eine Chloracetylgruppe bedeuten.